# EUROPEAN PATENT APPLICATION

(11) **EP 1 918 714 A1**
(43) Date of publication of application: **07.05.2008**
(21) Application number: 06123337.5
(22) Date of filing: 02.11.2006
(51) Int. Cl.: G01N 33/68

(54) **Compounds and methods for double labelling of polypeptides to allow multiplexing in mass spectrometric analysis**

(71) Applicant: Koninklijke Philips Electronics N.V., 5621 BA Eindhoven (NL)
(72) Inventor: The designation of the inventor has not yet been filed
(74) Representative: van Wermeskerken, Stephanie Christine

(57) **Abstract**

The present invention describes double labelling reagents with both an isotopic and isobaric label component suitable for differentially labelling different - protein samples. After labelling of the individual protein samples, all samples are pooled. Peptides from the pooled samples are isolated and analysed by mass spectrometry for determining the relative concentration of each differentially double-labelled polypeptide.

## Description

### FIELD OF THE INVENTION

The present invention provides methods and tools for the screening of targets by mass spectrometric approaches. More particularly the methods and tools of the invention allow the parallel screening of multiple samples using liquid chromatography mass spectrometry using a combination of isotopic and isobaric peptide labelling procedures.

### BACKGROUND OF THE INVENTION

The analysis and identification of proteins from highly complex mixtures demands tremendous resolving power. Two methods commonly used to resolve such mixtures are 2-Dimensional Gel Electrophoresis (2D-GE) and (2-Dimensional) Liquid Chromatography ((2D)-LC).

High-resolution 2D-GE was introduced in by Klose (1975) Humangenetik 26, 231-243 and O'Farrell (1975), J. Biol. Chem. 250, 4007-40021. 2D-GE is unsurpassed in resolution (>5000 proteins), but suffers from lack of automation and reproducibility. Furthermore, proteins such as hydrophobic membrane proteins, basic proteins, acidic proteins, very large or very small proteins are poorly resolved.

For the many promising disease markers which escape identification using gel electrophoresis, 2D-LC provides a good alternative, allowing high-resolution separation of complex protein mixtures [Hanash et al. (2003) Nature 422, 226-232; Lipton et al. (2002) Proc. Natl. Acad. Sci. USA 99, 11049-11054]. 2D-LC approaches are more suitable than 2D-GE to separate smaller proteins and the automation and throughput is significantly better. Several technologies to separate protein/peptide digests by liquid chromatography have been described, including capillary electrophoresis (CE), reversed-phase (RP) HPLC, and 2-dimensional liquid chromatography.

Typically, peptides and proteins isolated by 2D-GE or 2D-LC are identified either by mass spectrometry or by determining amino acid composition/and or amino acid sequence.

The most relevant problem in determining statistically significant protein expression differences between different samples (e.g. disease vs. control) by mass spectrometry technologies is the relative quantitation of MS signals from different samples. Therefore, it is highly desirable to develop methodologies, which allow the processing of multiple samples in parallel in order to reduce the technical variability between different samples as much as possible.

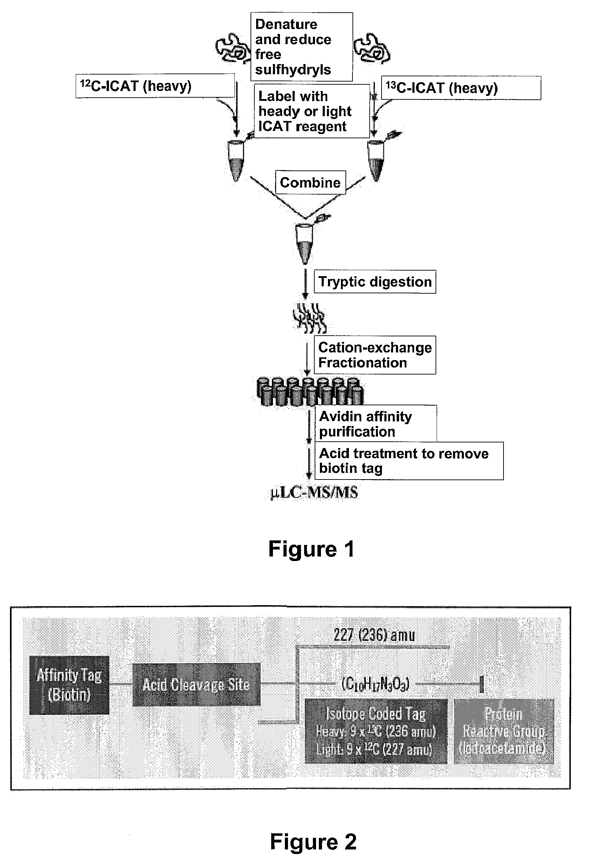

Recently, two approaches have been introduced to achieve this. The first technique is called Isotope-Coded Affinity Tag (ICAT) technology and allows for quantitative proteomic analysis based on differential isotopic tagging of related protein mixtures [Gygi et al. (1999) Nat. Biotechnol. 17, 994-999]. The ICAT reagent described uses three functional elements: a thiol-reactive group for the selective labelling of reduced cysteine residues, a biotin affinity tag to allow for selective isolation of cysteine labelled peptides, and an isotopic tag, which is synthesized in two isotopic forms, the "light" (non-isotopic) or "heavy" (utilizing ²H or ¹³C) form. As only a moderate number of generated peptides contains cysteine in its primary sequence, the complexity of the digested affinity purified sample decreases dramatically. Two individually labelled peptide samples ("light" vs. "heavy") are combined before they are applied to separation techniques like liquid chromatography (Figure 1) followed by MS identification.

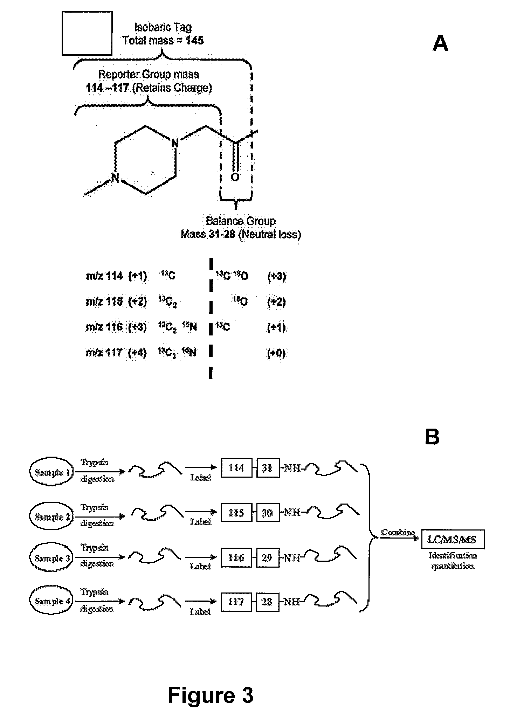

An alternative approach makes use of isobarically labelled reagents referred to as Isobaric Tag for Relative and Absolute Quantitation (iTRAQ). This technique described by Ross et al. (2004, Mol. Cell. Proteomics 3, 1154-1169) provides for four different iTRAQ reagents each containing a reporter group, a balance group and a peptide reactive group which reacts with primary amine groups (Figure 3). The reporter group is a group with a mass of 114, 115, 116 or 117 Da, depending on differential isotopic combinations of ¹²C/¹³C and ¹⁶O/¹⁸O in each reagent. The balance group varies in mass from 31 to 28 Da to ensure that the combined mass of the reporter group and the balance group remains constant (145 Da) for the four reagents. Accordingly, labelling of the same peptide with each of these reagents (comprising specific combinations of different isotopes in reporter and balance group) results in peptides which are isobaric and co-elute in liquid chromatography and consequently are chromatographically indistinguishable. During MS/MS analysis, the reporter group ions fragment from the peptides, displaying distinct masses of 114 to 117 Da. The intensity of these fragments can be used for quantitation of the individual peptides. Accordingly the presence of a particular peptide in four different samples can be differentially determined by labelling each of the samples with a different iTRAQ reagent. iTRAQ labels are used to quantitate the relative as well as the absolute peptide abundance from MS/MS spectra and allows labelling of up to four different samples within a single experiment. In addition, the labelled peptides are isobaric and all contribute to one ion species that is observed in the MS and that is used for CID. This results in increased signal intensity and an increased probability of correct peptide identification, particularly for molecules with low abundancy, which is a characteristic of many biologically meaningful proteins. Recently, the set of commercially available ITRAQ reagents has grown to eight, including reporter groups with a Mass of 113, 118, 119 and 121.

Absolute and relative quantitation of MS signals is an unsolved issue in biomarker discovery by technologies based on mass spectrometry approaches. However, the identification of relevant expression differences between different samples, e.g., (different disease groups, different progression stages of a disease, disease vs. control/healthy) needs highly reproducible techniques, as relevant biomarkers can only be derived from a multitude of measurements.

The above described ICAT and iTRAQ techniques allow the multiplexing of either 2 (ICAT) or up to 4 or 8 (iTRAQ) individual samples and allows to process them simultaneously thereby minimising technical variability. Nevertheless, both these approaches have significant limitations. The ICAT approach only allows the investigation of two different samples, or two disease groups, which normally does not represent the full spectrum of disease relevant groups (e.g., different stages of progression of a disease). Therefore, it would be very advantageous to have a technique available, which allows the investigation of more than 2 groups. The iTRAQ technology, while allowing multiple sample analysis has the clear disadvantage that it requires performing MS/MS scans on each MS signal (for both unlabelled and labelled peptides) for relative quantitation of the reporter signals. In practice, this is not feasible when the starting material is of high complexity such as in human serum, or other body fluids, as the analysis will be very time consuming.

### SUMMARY OF THE INVENTION

The present invention overcomes the limited multiplexing of the prior art techniques by combining an isotopic label component and an isobaric label component in one single labelling reagent. By generating a greater number of differential labelling reagents that can be used, the multiplexicity of the combined sample detection in increased.

The labelling tools and methods according to the present invention have the advantage that the differentially labelled peptides can be easily identified on MS, thereby limiting the analysis to the peptides of interest. The use of double labelling methods of the present invention have the advantage that the analysis time by mass spectrometry is significantly reduced, because only those peptides need to be analysed by MS/MS for which a differential expression of a peptide is observed between different samples.

The tools and methods making use of the labelling method of the present invention are of interest for multiple screening of protein samples. More particularly, they make it possible to simultaneously determine quantitative and/or qualitative differences of proteins in different protein samples. This can be used to determine differences in expression levels, and also allows the determining of differences in proteolytic processing between different protein samples, e.g. in the context of disease. In this regard the present invention provides methods for multiple screening of different samples which can be directly compared, e.g. in the comparison of different stages of a disease and/or different disease forms.

The labelling reagents of the present invention allow to perform multiplexing labelling experiments wherein only one labelling step is used. While the final identification of all of the differentially labelled proteins is ensured on MS/MS, the presence of an isotopic label on the proteins or peptides allows the identification of all of the differentially labelled peptides in a MS spectrum as they will appear as distinct separated peaks, with a mass difference corresponding to difference in the isotope label. This allows the limitation of the further MS/MS analysis to those peptides which have been identified as being differentially expressed in the MS step. In addition, the presence of an affinity label allows further limitation of the first MS analysis to effectively labelled peptides.

Particular and preferred aspects of the invention are set out in the accompanying independent and dependent claims. Features from the dependent claims may be combined with features of the independent claims and with features of other dependent claims as appropriate and not merely as explicitly set out in the claims.

A first aspect of the invention relates to a set of labelling reagents for mass spectrometry analysis of polypeptides, each labelling reagent comprising an allobaric label component, a protein/peptide reactive group, and an isobaric label component comprising a reporter group (RP) and a balance group (BG).

In one embodiment the allobaric label component is an isotopic label component, which is not comprised within the isobaric label component in the labelling reagent. Alternatively, the allobaric label component is comprised within the reporter and/or the balance group of the isobaric label in the labelling reagent.

In one embodiment of the labelling reagents of the present invention, the protein/peptide reactive group reacts with a cysteine residue in a protein or polypeptide.

Particular embodiments of the present invention provide labelling reagents as described above, further comprising an affinity tag, which is optionally cleavable. In a further particular embodiment, the affinity tag is biotin, or a molecule derived therefrom.

A further aspect of the invention provides methods for simultaneously analysing the presence of one or more polypeptides comprising a functional group in different samples, which comprise a labelling step, wherein each sample is labelled with one of a set of differential double labelling reagents according to the present invention having the same or essentially the same chemical structure and comprising an allobaric and an isobaric label component. The labelling step is ensured by allowing the protein reactive group of the labelling reagents to react with a functional group of the one or more polypeptides in the samples, or peptides generated therefrom (as a result e.g. of proteolytic cleavage). Further steps of the methods according to this aspect of the invention include, but are not limited to (2) the pooling of the different samples to obtain a polypeptide sample mix, (3) the selective isolation of the double labelled polypeptides or peptides from the polypeptide sample mix, and (4) the analysis of the relative occurrence of the isolated labelled polypeptides or peptides by Mass Spectroscopy,

The set of labelling reagents used in the methods of the present invention typically comprises a unique combination of an allobaric and an isobaric label component. According to one embodiment the allobaric labelling components are isotopic labelling components.

According to a particular embodiment, the labelling reagents used in the above-described methods comprise an affinity tag.

According to one embodiment the methods of the invention further comprise, after step (a); a cleaving step, whereby the proteins or polypeptides in the sample are cleaved into peptides. In a particular embodiment this cleaving is performed with trypsin.

According to a particular embodiment of this aspect of the invention, step c) comprises selectively isolating the polypeptides or peptides based on an affinity tag present on the labelled polypeptides or peptides.

In a particular embodiment of this method, the functional group present on the polypeptides or peptides in the sample and which reacts with the protein reactive group of the labelling reagents is a thiol. 1.

According to another embodiment of this method, the functional group present on the polypeptides or peptides in the sample which reacts with the protein reactive group of the labelling reagents is present as a result of a modification of another functional group. Accordingly the methods of the present invention optionally comprise an additional modification step, in which the polypeptides or peptides in a sample are modified so as to obtain a functional group suitable for reacting with the protein reactive group of a set of labelling reagents as described herein.

According to one embodiment of the methods of this aspect of the invention, step (c) additionally or alternatively comprises the step of isolating a labelled polypeptide or peptide fraction from these pooled polypeptides by electrophoresis or chromatography.

According to one embodiment of the methods of this aspect of the invention, step (d) comprises the step of determining with MS the relative amount of different masses in the isolated labelled polypeptide or peptide fraction obtained in step (c). Optionally, in the methods of this aspect of the invention, step (d) further comprises the step of subjecting this isolated labelled polypeptide fraction to collision-induced dissociation (CID) and determining the relative amount of different isobarically labelled polypeptides therein. In specific embodiments, the relative amount of different isobarically labelled polypeptide is determined by determining the relative amount of reporter groups released from the isobaric label after CID.

According to one embodiment the methods of this aspect of the invention further comprise the step of determining the sequence of this polypeptide.

A further aspect of the invention relates to a method for labelling a polypeptide sample with a labelling reagent as described in the first aspect of the invention having an isobaric and an allobaric label component comprising the step of reacting this labelling reagent with a functional group on the polypeptides in the sample.

A further aspect of the invention relates to the use of the methods as described above for identifying proteins which are differentially expressed in different biological or experimental protein samples, such as e.g. a control individual and one or more protein samples of different disease conditions, different disease stages, different subjects having or suspected to have the same disease...etc.

Yet a further aspect of the present invention provides mixtures of polypeptides or peptides originating from different samples, wherein each polyeptide or peptide from a particular sample comprises a label comprising an isotopic label component and isobaric label component, which label has been linked to the polypeptides or peptides through a functional group in the polypeptides or peptides.

In one embodiment, the functional group of the polypeptides or peptides of the mixture of the invention to which the label has been linked is a functional group selected from the group consisting of a primary amine on the N-terminus or on Lysine, a carboxylgroup on Aspartic acid, Glutamic acid or the C-terminus and a thiol group on Cysteine.

In a particular embodiment, this mixture of peptides is a mixture of tryptic peptides of biological proteins resulting from a tryptic digest of a protein sample.

In a particular embodiment, the number of different labels present on the peptides or polypeptides in the mixture of this aspect of the invention is at least 4.

A further aspect of the invention provides kits comprising four or more labelling reagents comprising an isobaric label component, an allobaric label component and a protein/peptide reactive group, wherein each labelling reagent in this kit has the same or essentially the same chemical structure and wherein each labelling reagent comprises a unique combination of the allobaric and the isobaric label component.

In one embodiment, the labelling reagents in this kit comprise an affinity tag.

A further aspect of the present invention provides methods for determining relative amounts of individual polypeptides or peptides of a different sample having the same amino acid sequence, within a pooled mixture of these polypeptides or peptides, wherein each polypeptide or peptide from a different sample is, prior to pooling, labelled with one of a set of differential double labelling reagents, whereby the set of differential double labelling reagents used have the same or essentially the same chemical structure and carry an isotopic and an isobaric label component and each labelling reagent comprises a unique combination of different isotopic and an isobaric label components, comprising the steps of:
a) optionally isolating all labelled polypeptides or peptides by liquid chromatography, so as to obtain an isolated fraction comprising said individual polypeptides or peptides having the same amino acid sequence
b) separating the isolated fraction of polypeptides or peptides obtained in step (a) into further fractions of polypeptides or peptides each with different mass, by a first MS and determining the relative amount of each fraction with a different mass.
c) subjecting the peptide fractions obtained in step (b) to a second MS under conditions wherein the isobaric label component (and optionally the polypeptide or peptide) is fragmented and determining the amounts of each fragmented isobaric label component.
d) determining, from the relative amount of each of the isobaric label components determined in step (c), the relative amount of the individual peptides within a polypeptide or peptide fraction obtained in step (b), and
e) calculating from the relative amounts of each polypeptide or peptide fraction with a different mass determined in step (b) and from the relative amounts of the individual peptides within a peptide fraction obtained in step (d), the relative amount of each labelled individual polypeptide having the same amino acid sequence, present in said pooled mixture.

Yet a further aspect of the present invention relates to a device (100) for multiplex labelling and analysis of protein samples comprising at least two sources of samples (101), a labelling unit (103) and corresponding label sources (104) each comprising a labelling reagent comprising an allobaric label component, a protein/peptide reactive group, and an isobaric label component comprising a reporter group (RG) and a balance group (BG), and further comprising a separation unit (107), a mass spectrometer unit (108) and a data analysis unit (109).

In a particular embodiment this device further comprises one or more elements selected from the group consisting of a sample preparation unit (102), a protein cleavage unit (105) and an affinity purification unit (106).

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference Figures quoted below refer to the attached drawings.

### BRIEF DESCRIPTION OF THE FIGURES

Fig. 1 shows a proteomic flow-chart for quantitative analysis of protein profiles with cleavable ICAT reagents (taken from Li et al. (2003) Mol. Cel. Proteom. 2, 1198-1204)
Fig. 2 shows a schematic structure of a cleavable ICAT reagent of the prior art.
Fig. 3 shows exemplary structures of iTRAQ labels (A) and peptides labelled therewith (B). The label consists of a reporter group with a mass ranging from 114 to 117 Da and a balance group with a mass ranging from 31 to 28 Da.
Fig. 4 shows, in accordance with a particular embodiment of the invention, a label whereby the isotopic label component is introduced within the isobaric label component, i.e. by replacing an oxygen atom in the balance group (Figure 4A) by a sulphur (Figure 4B). In Figure 4C, the isotopic label component is introduced on the propyl side chain of the piperazine group of the reporter group of the isobaric label component.
Fig. 5 shows a non-limiting set of examples of schematic representations of labelling reagents according to particular embodiments of the present invention.

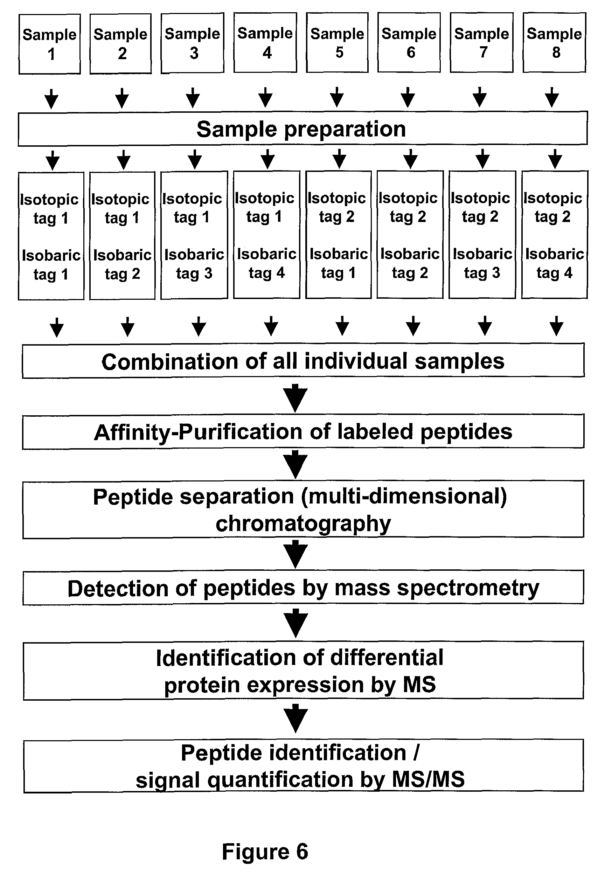

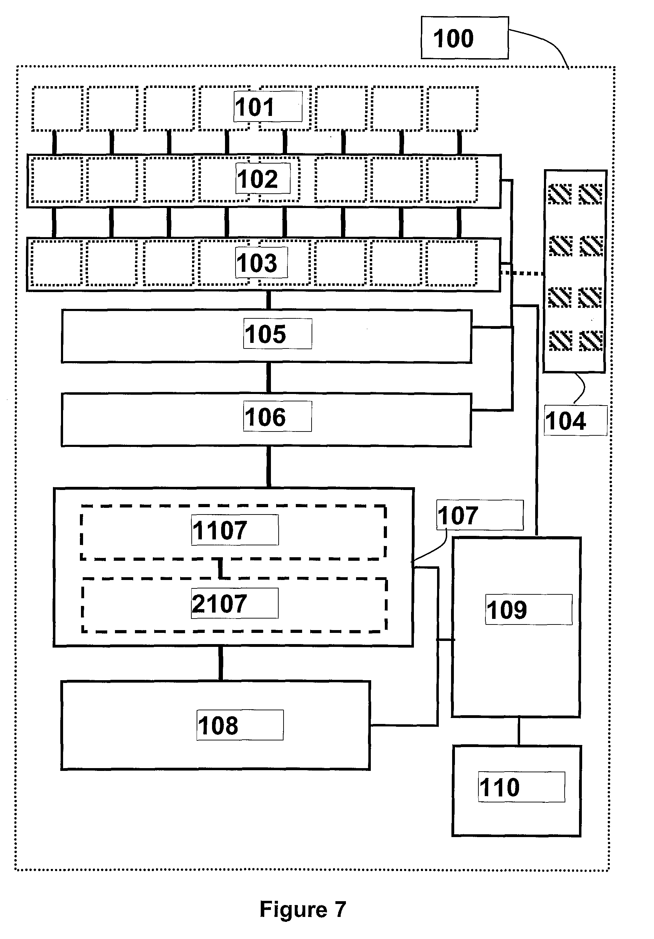

Fig. 6 demonstrates the simultaneous analysis of multiple samples using labelling reagents comprising both and isotopic and an isobaric label component in accordance with particular embodiments of the present invention. Figure 7 shows in accordance with a particular embodiment of the present invention a device for multiplex analysis of 8 protein samples (100), comprising at least two sample sources (101), a sample preparation unit (102), a labelling unit (103) with labelling reagent source (104), a protein cleavage unit (105) an affinity-purification unit (106) (e.g. avidin affinity chromatography system), a separation unit (107) comprising two consecutively coupled separation systems (1107) and (2107), a mass spectrometer unit (108) and a control and analysis circuitry and data analysis unit (109) coupled to a read out system (110).

In the different Figures, the same reference signs refer to the same or analogous elements.

### DETAILED DESCRIPTION OF THE EMBODIMENTS

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The following terms or definitions are provided solely to aid in the understanding of the invention. These definitions should not be construed to have a scope less than understood by a person of ordinary skill in the art.

The term "polypeptide" or "protein", as used herein, refers to a plurality of natural or modified amino acids connected via a peptide bond. The length of a polypeptide can vary from 2 to several thousand amino acids (the term thus also includes what is generally referred to as oligopeptides). Included within this scope are polypeptides comprising one or more amino acids which are modified by *in vivo* posttranslational modifications such as glycosylation, phosphorylation, etc. and/or comprising one or more amino acids which have been modified *in vitro* with protein modifying agents (e.g. alkylating or acetylating agents).

The term "polypeptide fragment" or "peptide" as used herein is used to refer to the amino acid sequence obtained after cleavage of a protein or polypeptide. A polypeptide fragment or peptide is not limited in size or nature.

The terms "internal", "aminoterminal" and "carboxyterminal" when referring to a peptide are used herein to refer to the corresponding location of a peptide in a protein or polypeptide. For example, in a tryptic cleavage of protein NH₂-X₁-K-X₂-R-X₃-K-X₄-COOH (wherein X₁, X₂, X₃ and X₄ are peptide sequences of indifferent length without Lysine (K) or Arginine (R)), the aminoterminal peptide is NH₂-X₁-K-COOH, the internal peptides are NH₂-X₂-R-COOH and NH₂-X₃-K-COOH and the carboxyterminal peptide is NH₂-X₄-COOH.

The term "protein cleavage" as used herein relates to the hydrolysis of a peptide bond between two amino acids in a polypeptide. This includes both chemical or enzymatic hydrolysis.

The term "fragmentation" as used herein refers to the breaking of one or more chemical bonds and subsequent release of one or more parts of a molecule as obtained e.g. by collision-induced dissociation (CID) in Tandem Mass spectrometry (MS) or MS/MS analysis. In certain embodiments the bond is a peptide bond, but it is not limited thereto.

The term "label" as used herein refers to a compound which is covalently linked to a peptide or polypeptide and which, based on its particular properties is detectable on a mass spectrometer. Where the label can be covalently bound to a peptide or polypeptide, this is ensured by a protein/peptide reactive group (PRG). According to the present invention one label can comprise more than one "label component", i.e. more than one component which based on its particular properties is differentially detectable.. Generally the term "label" will be used to refer to the molecule as present on the polypeptide or peptide, and the term "labelling reagent" is used to refer to the unbound label (or the combination of compounds used for ensuring the presence of the label on the protein or peptide), prior to reaction with a protein or peptide, i.e. still comprising a free protein/ peptide reactive group.

The terms "double label" and "double labelling reagent" as used herein refer to labels or reagents comprising both an allobaric (e.g. isotopic) and an isobaric label component. Accordingly, the term "double labelled" when referring to a peptide or protein refers to the presence of a label comprising both label components on the peptide or protein.

The term "isotopic label" as used herein refers to a label comprising one or more isotopes of an atom.

The term "allobaric label components" as used herein refers to a set of molecules with essentially the same structure and behaving in the same way in electrophoresis and chromatography, but differing in one or more atoms to generate a difference in mass, and which can be used as (part of) a label. Accordingly, a set of labelling reagents or labels comprising a set of allobaric label components will differ in mass. Where the difference in the one or more atoms corresponds to the fact that the label components comprise different isotopes of the same atom, reference is made to "isotopic label components".

The term "isotopic label components" as used herein refers to a set of molecules with essentially the same structure and behaving in the same way in electrophoresis and chromatography, but differing in one or more isotopes to generate a difference in mass, and which can be used as (part of) a label. Identical peptides each labelled with a label comprising a label component with the same or essentially the same chemical formula, but differing in mass based on the presence of different isotopes of the same atoms (either in number or type) can be distinguished from each other in MS.

The term "isobaric label components" as used herein refers to a set of molecules with the same structure, and the same mass, which upon fragmentation release a particular fragment with the same structure which differs in mass between the different isobaric label components of a set, due to a differential distribution of isotopes within the isobaric label component. An isobaric label component typically comprises a reporter group (RG) and a balance group (BG), which are separated upon fragmentation and which display a differential distribution of isotopes. The "combined mass" of a set of isobaric label components refers to the total mass of the reporter group and the balance group for that set of isobaric label components.

The term "reporter group (RG)" refers to the part of isobaric label component which generates fragment ions upon CID, which individually differ in mass within a set of isobaric label components, as a result of a differential occurrence of one or more isotopes. For example, different reporter groups within a set of isobaric label components are characterized by the differential occurrence of ¹²C/¹³C and/or ¹⁶O/¹⁸O in the reporter group. The typical fragments generated upon release of the reporter group are used quantitatea polypeptide or peptide labelled with a label comprising an isobaric label component. Typically the fragment ions of the reporter group appear in the low-mass region of an MS spectrum, where other fragment ions are not generally found.

The term "balance group (BG)" as used herein refers to the part of isobaric label component, which contains a certain compensating number of isotopes so as to ensure that the combined mass of the reporter group and balance group is constant for the different isobaric label components. The balance group may or may not be released from the label upon CID.

The term "functional group" as used herein refers to a chemical function on an amino acid, which can be used for binding (generally, covalent binding) to a chemical compound. Functional groups can be present on the side chain of an amino acid or on the aminoterminus or carboxyterminus of a polypeptide or peptide. The term encompasses both functional groups which are naturally present on a peptide or polypeptide and those introduced via e.g. a chemical reaction using protein-modifying agents.

The term "protein/peptide reactive group (PRG)" as used herein refers to a chemical function on a compound (e.g. a labelling reagent) that is capable of reacting with a functional group on an amino acid of a protein or peptide resulting in the binding (noncovalent or covalent) of such compound to the amino acid.

The present invention relates to labelling reagents for use in the comparison of different protein samples comprising both an isotopic and isobaric label component, thereby allowing the generation of a larger number of differential labels for the differentiation of similar peptides of different origin within one pooled polypeptide or peptide mixture. Using the differential labelling reagents of the invention it is moreover possible to identify the presence and the relative amount of a protein or a peptide in a pooled sample.

Accordingly, the methods and tools of the present invention are of particular interest in the analysis of a set of samples for which a comparable analysis is of interest. Such a set of samples can be, but is not limited to, samples from a patient taken at different time points, samples of different clinical versions of a disease, samples of different patients etc.. The present invention thus provides methods and tools for identifying markers of disease progression, for differential diagnosis, and moreover for multiplex analysis in biochemical or physiological assays.

The methods and tools of the present invention relate to the analysis of protein samples. The term 'sample' as used herein is not intended to necessarily include or exclude any processing steps prior to the performing of the methods of the invention. The samples can be rough unprocessed samples, extracted protein fractions, purified protein fractions etc... As indicated above, the proteins present in the samples. According to one embodiment the protein samples are pre-processed by immunodepletion of abundant proteins.

Protein samples which are suitable for analysis with the labelling reagents and methods of the present invention include samples of viral, prokaryote, bacterial, eukaryote, fungal, yeast, vegetal, invertebrate, vertebrate, mammalian and human origin. The preparation of samples differs depending on the organism, tissue or organ investigated, but standard procedures are usually available and known to the expert. With respect to mammalian and human protein samples it covers the isolation of cultured cells, laser micro-dissected cells, body tissue, body fluids, or other relevant samples of interest. With respect to the fractionation of proteins in a sample, cell lysis is the first step in cell fractionation and protein purification. Many techniques are available for the disruption of cells, including physical, enzymatic and detergent-based methods. Historically, physical lysis has been the method of choice for cell disruption; (homogenisation, osmotic lysis, ultrasound cell disruption) however, it often requires expensive, cumbersome equipment and involves protocols that are sometimes difficult to repeat due to variability in the apparatus (such as loose-fitting compared with tight-fitting homogenisation pestles). In recent years, detergent-based lysis has become very popular due to ease of use, low cost and efficient protocols.

Mammalian cells have a plasma membrane, a protein-lipid bilayer that forms a barrier separating cell contents from the extracellular environment. Lipids comprising the plasma membrane are amphipathic, having hydrophilic and hydrophobic moieties that associate spontaneously to form a closed bimolecular sheet. Membrane proteins are embedded in the lipid bilayer, held in place by one or more domains spanning the hydrophobic core. In addition, peripheral proteins bind the inner or outer surface of the bilayer through interactions with integral membrane proteins or with polar lipid head groups. The nature of the lipid and protein content varies with cell type. Clearly, the technique chosen for the disruption of cells, whether physical or detergent-based, must take into consideration the origin of the cells or tissues being examined and the inherent ease or difficulty in disrupting their outer layer(s). In addition, the method must be compatible with the amount of material to be processed and the intended downstream applications.

In particular embodiments, protein extraction also includes the pre-fractionation of cellular proteins originated from different compartments (such as extracellular proteins, membrane proteins, cytosolic proteins, nuclear proteins, mitochondrial proteins). Other pre-fractionation methods separate proteins on physical properties such as isoelectric point, charge and molecular weight.

According to a particular embodiment, the samples are pre-treated prior to labelling or cleavage, so as to denature the proteins for optimised access to reagents or proteases, using appropriate agents (e.g., guanidinium chloride, urea, acids (e.g. 0,1 % trifluoric acid), bases (e.g. 50 % pyridine) and ionic or non-ionic detergents).

Furthermore, as will be detailed below, depending on the type of protein reactive groups in the labelling agent, the methods of the present invention envisage that the functional groups are irreversibly or reversibly modified with protein modification agents, prior to the labelling methods of the invention. Examples are the release of blocked aminotermini, the reduction of Cystine to Cysteine, blocking the functional thiol group of cysteine, acetylating the amine group of Lysine, etc....

The methods of the present invention thus optionally comprise a pre-treatment of the samples, which can be performed in a pre-treatment step comprising one or more of the sample preparation methods listed above. Accordingly, devices suitable for the methods of the present invention optionally comprise a sample preparation unit comprising one or more devices suitable for sample preparation e.g. sonication devices, chromatography systems (affinity, gelfiltration), ultrafiltration units, centrifuges, temperature controlled reaction vials with delivery systems for buffers, enzymes, detergents etc...

According to the present invention, tools and methods are provided which allow multiple labelling with only one labelling step. This is ensured, according to the present invention, by combining two types of label components suitable for differential detection in MS, in one labelling reagent, thereby ensuring a set of differential labelling reagents based on combinations of label components.

According to one embodiment, the labelling reagents of the invention comprise one label component which is from a set of isobaric (same mass) label components, and one which is from a set of allobaric (different masses) label components.

According to the present invention, the allobaric label components of the labelling reagents, are components which, differ in mass from each other, as a result of which the labelling reagents comprising them, will differ in mass. Accordingly, these components contribute a different mass to the peptides/proteins to which they are bound. Nevertheless, the allobaric label components of the present invention have essentially the same effect on the chromatographic or electrophoretic behaviour of the peptides or proteins to which they are bound.

According to one embodiment, the allobaric label components are components with essentially the same chemical structure, but wherein one atom is replaced by another in such a way that the structure of the label is minimally modified. Accordingly, labelling of identical peptides with a set of labelling reagents comprising such allobaric label components, will result in the peptides with slight (predetermined) differences in mass (which can thus be identified in MS) but which do not differ in e.g. chromatographic or electrophoretic behaviour prior to MS analysis.

According to an alternative embodiment, the allobaric label component of the labelling reagents of the invention is an isotopic label component, i.e. ensuring the difference in mass between different reagents of a set by a differential isotope composition.

Accordingly, in this embodiment, the labelling reagents of the present invention comprise an isotopic label component, an isobaric label component and a protein reactive group. Optionally, the labelling reagents further comprise an affinity tag. Generally, the isotopic label component and the isobaric label component make up two separate parts of the labelling reagents. However, as is described below, the isotopic label component can also be incorporated within the isobaric label component.

The combination of different isotopic and isobaric label components in the labelling reagents of the present invention allows a higher number of differential labelling reagents within one set, i.e. for use in the differential labelling of samples whereby after pooling of the samples, the origin of the sample can be identified. For instance, where the number of different isotopic label components is 2, and the number of isobaric label reagents is 4, a total of 2x4=8 combinations of label components, and thus of differential labelling reagents can be made. In this example, the set of labelling reagents will comprise 2 groups of 4 labelling reagents, whereby the 2 groups differ from each other in mass (due to the differential isotopic labelling), and whereby within each group, 4 labelling reagents will be isobaric, i.e. having an identical mass, but generating, in MS/MS, a reporter group with a different mass. Accordingly, identical peptides of different origin labelled with this set of labelling reagents can be identified, first in MS, as two peaks corresponding to the two groups of isotopically labelled peptides, which, when further analysed in MS/MS, will each further generate 4 peaks, corresponding to the differential reporter groups of the isobaric label components.

According to one embodiment, the isotopic label components, used in the combined isotopic/isobaric labelling reagents of the present invention, comprise a structure wherein one or more of the atoms in the isotopic label component are substituted with a stable isotope, so as to generate one or more compounds with the same chemical formula, but which are isotopically distinguishable based on their difference in mass. For example, any one or more of the hydrogen, nitrogen, oxygen or sulphur atoms in the isotopic label component can be replaced with their isotopically stable isotopes ²H, ¹³C, ¹⁵N, ¹⁷O, ¹⁸O or ³⁴S, respectively. For example, hydrogen is substituted with deuterium or carbon ¹²C is substituted with ¹³C. Using one more of the above isotopes, 2, 3, 4, 5, 6, 7, 8, or an even higher number of isotopic label components can be generated, each having the same structure but with a different Mr. As indicated above, the difference in Mr of these label components, upon binding of each of the labelling reagents on identical polypeptides in a different sample, is reflected in the resulting Mr of the polypeptide or peptide generated therefrom.

Examples of groups which can function as isotopic label components by differential introduction of one or more isotopes include but are not limited to ethers, polyethers, ether diamines, polyether diamines, diamines, amides, polyamides, polythioethers, disulfides, silyl ethers, alkyl or alkenyl chains (straight chain, branched or with portions which are cyclic), aryl, diaryl or alkyl-aryl groups. In particular embodiments, aryl groups present in the isotopic component of the labelling reagents of the present invention contain one or more heteroatoms (e.g., N, O or S atoms).

According to a particular embodiment, the isotopic label component of the labelling reagent is such that it does not undergo peptide-like fragmentation during (MS)ⁿ analysis. To promote ionization, the isotopic label component, and more particularly the isotopic group therein, may contain groups or moieties such as acidic or basic groups, e.g., COOH, SO₃H, primary, secondary or tertiary amino groups, nitrogen-heterocycles, ethers, or combinations of these groups. In particular embodiments, the isotopic label component contains groups having a permanent charge, e.g., phosphonium groups, quaternary ammonium groups, sulfonium groups, chelated metal ions, tetralky or tetraryl borate or stable carbanions.

According to one embodiment, the structure of the isotopic label component of the labelling reagents of the present invention corresponds to the general formula -B¹-X¹-(CH₂)ₙ-[X₂-(CH₂)ₘ]ₓ-X₃-(CH₂)ₚ-X⁴-B²- wherein:
- X¹, X², X³ and X⁴, independently of one another, are selected from O, S, NH, NR, NRR'⁺, CO, COO, COS, S-S, SO, SO₂, CO-NR', CS-NR', Si-O, aryl or diaryl groups. (R is an alkyl, alkenyl, alkynyl, alkoxy or aryl group and R' is a hydrogen, an alkyl, alkenyl, alkynyl, alkoxy or aryl group). In particular embodiments X¹-X⁴ are absent, but typically at least one of X¹-X⁴ is present.
- n, m, p and q are whole numbers with values from 0 to about 100. In particular embodiments one of n, m, p or q is not 0 and x is also a whole number ranging from 0 to about 100 where the sum of n+xm+p+q is less than about 100 and in more particular embodiments less than about 20; and
- B¹ and B², independently of one another, are optional moieties that facilitate bonding of other parts in the labelling reagent (e.g. an affinity tag, or a protein/peptide reactive group described below) to the isotopic label component or that prevent undesired cleavage of those linkers from the isotopic label component and are selected, for example, from COO, CO, CO-NR', CS-NR' and optionally contains one or more CH₂ groups alone or in combination with other groups, e.g. (CH₂)_{q}-CONR', (CH₂)_{q}-CS-NR', or (CH₂)q,

In particular embodiments, one or more of the CH₂ groups of the isotopic label component having the structure described above, is optionally substituted with small (C₁-C₆) alkyl, alkenyl, or alkoxy groups, an aryl group or is substituted with functional groups that promote ionization, such as acidic or basic groups or groups carrying permanent positive or negative charge. In particular embodiments, one or more single bonds connecting CH₂ groups in the linker are replaced with a double or a triple bond. In particular embodiments R and R' alkyl, alkenyl, alkynyl or alkoxy groups are small having 1 to about 6 carbon atoms.

According to a particular embodiment, the isotopic label components of the present invention are made up of one or two atoms which are either incorporated in or a side chain of another part of the labelling reagents, most particularly of the isobaric label component. This embodiment will be described more in detail below.

The labelling reagents of the present invention, in addition to an allobaric or isotopic label component described above, further comprise an isobaric label component.

According to one embodiment, the isobaric label component comprises a reporter group (RP) and a balance group (BG), whereby the mass of the reporter group is specific for each isobaric label component (within a set of labelling reagents), and the overall mass of the reporter group and the balance group of the different isobaric label components are kept constant.

According to a particular embodiment, the general structure of the isobaric label component can be represented by the general formula RP-X'-BG-Y'-. whereby the reporter group (RP) is a terminal part of the isobaric label component, and typically also of the labelling reagents (see below). X' and Y' both represent a bond or link. According to an alternative embodiment, the reporter group is internal in the isobaric label component, and the general structure of the isobaric label component within the labelling reagents can be represented by the general formula -X'- RP-X'-BG-Y'-.

The concept of isobaric labelling is exemplified in Figure 3. Figure 3A provides an example of an isobaric label component. In this embodiment, the isobaric label component consists of a reporter group based on *N*-methylpiperazine, and a mass balance group which is a carbonyl. In Figure 3B, the isobaric label component is bound directly to a peptide through a peptide-reactive group which is an NHS ester. While the mass of the reporter group is specific for each isobaric label component within a set, the overall mass of the reporter group and the balance group of the different isobaric label components are kept constant.

According to a particular embodiment the differential mass of the reporter group in the different isobaric label components of a set of labelling reagents is ensured by using differential isotopic enrichment with ¹³C, ¹⁵N, and ¹⁸O atoms as indicated, to avoid problems with chromatographic separation seen with enrichment involving deuterium substitution. In view of the identical structure of the different isobaric label components, the number and position of enriched centers in the ring has no effect on chromatographic or MS behaviour.

The isobaric label component can thus be bound directly to the peptide or to the isotopic group through an amide linkage. In Figure 3, the isobaric label component is flanked by a protein reactive group, which is an amine specific reactive group. Upon reaction of the amine specific reactive group of this labelling reagent with a peptide, the label becomes connected via an amide linkage to amine functional groups (N-terminal or amine group of lysine). These amide linkages fragment in a similar fashion to backbone peptide bonds when subjected to CID. Other suitable methods to fragment peptides include CAD (collisionally activated dissociation), ETD (electron transfer dissociation), ECD (electron capture dissociation), IRMPD (infrared multiphoton dissociation) and BIRD (blackbody infrared radiative dissociation. Following fragmentation of the amide bond when subjected to CID, the balance (carbonyl) moiety is lost (neutral loss), while charge is retained by the reporter group fragment.

In Figure 3A, the numbers in parentheses indicate the number of enriched centres in each section of the molecule. Part B of Figure 3 illustrates the labelling with four isobaric combinations comprising four different reporter group masses. A mixture of four identical peptides each labelled with one member of the multiplex set appears as a single, unresolved precursor ion in MS (identical m/z). Following CID, the four reporter group ions appear as distinct masses (114-117 Da). All other sequence-informative fragment ions (b-, y-, etc.) remain isobaric, and their individual ion current signals (signal intensities) are additive. The relative concentration of the peptides can thus be deduced from the relative intensities of the corresponding reporter ions. When using the labelling reagents of the present invention, quantitation is thus performed at the MS/MS stage rather than in MS.

As indicated above, the reporter group RP of an isobaric label component is a group that has a unique mass (or mass to charge ratio) that can be determined. Accordingly, each reporter group of a set of isobaric label components (or the corresponding labelling reagents) has a unique mass. In particular embodiments, each reporter group of a set of isobaric label compoents comprises one or more heavy atom isotopes to achieve the unique mass. For example, isotopes of carbon (¹²C, ¹³C and ¹⁴C), nitrogen (¹⁴N and ¹⁵N), oxygen (¹⁶O and ¹⁸O) or hydrogen (hydrogen, deuterium and tritium) can be used in the preparation of differing reporter groups. Examples of stable "heavy" atom isotopes suitable for use in the reporter group include ¹³C, ¹⁵N, ¹⁸O and deuterium. These are not limiting as other light and heavy atom isotopes are also suitable for incorporation in the reporter group. Basic starting materials suitable for preparing reporter groups comprising light and heavy atom isotopes are available from various commercial sources such as Cambridge Isotope Laboratories and Isotec. According to one embodiment, the reporter group ranges in mass from m/z 114.1 to 117.1, while the balance group ranges in mass from 28 to 31 Da, such that the combined mass remains constant (145.1 Da) for each of the four reagents.

According to the present invention, the unique reporter group of the isobaric label component of the labelling reagents of the present invention is used to identify and quantitate the peptides in different samples. In a particular embodiment, the unique reporter group remains present as part of the label on one or multiple polypeptides of a sample. In this way information about the reporter group is associated with information about the labelled polypeptides of the sample.

Alternatively, however, as in the Example described above, the reporter group is removed from the labelled polypeptide prior to its identification. Thus, the reporter group need not be physically linked to a (poly)peptide at the moment when the reporter is determined.

Indeed, according to this embodiment, the unique mass of the reporter is, for example, determined in a second mass analysis of a tandem mass analyzer, after ions of a labelled (poly)peptide are fragmented to produce daughter fragment ions of the (poly)peptide and detectable reporter groups. The particular reporter group is used to identify the sample from which a particular (poly)peptide originated. Further, the determined quantity of the unique reporter group, either relative to the amount of other reporter groups or relative to a calibration standard (e.g. a polypeptide labelled with a specific reporter group), is optionally used to determine the relative or absolute amount (often expressed as a concentration and/or quantity) of polypeptide in the sample or samples. Therefore the reporter group that is present in the isobaric component of each labelling reagent used to label each particular sample provides different types of information, such as the amount of one or more polypeptides in a particular sample.

The reporter group either comprises a fixed charge or is capable of becoming ionised. Accordingly, the labelling reagent comprising the isobaric label component is used to label the reactive polypeptide in a salt or zwitterionic form. Ionisation of the reporter facilitates its determination in a mass spectrometer.

Accordingly, in particular embodiments the reporter group is determined as an ion, sometimes referred to as a "signature ion". When ionised, the reporter comprises one or more net positive or negative charges. For example, the reporter group comprises one or more basic nitrogen atoms (positive charge) or one or more ionizable acidic groups such as a carboxylic acid group, sulfonic acid group or phosphoric acid group (negative charge). Non-limiting examples of reporter groups comprising a basic nitrogen include substituted or unsubstituted morpholines, piperidines or piperazines.

In particular embodiments, the reporter group is a 5, 6 or 7 membered heterocyclic ring comprising a ring nitrogen atom that is N-alkylated with a substituted or unsubstituted acetic acid moiety to which the polypeptide is linked through the carbonyl carbon of the N-alkyl acetic acid moiety, wherein each different isobaric label comprises one or more heavy atom isotopes. This heterocyclic ring is substituted or unsubstituted. The heterocyclic ring is aliphatic or aromatic. Possible substituents of the heterocyclic moiety include alkyl, alkoxy and aryl groups. The substituents comprise protected or unprotected groups, such as amine, hydroxyl or thiol groups, suitable for linking the polypeptide to a support. In particular embodiments, the heterocyclic ring comprises additional heteroatoms such as one or more nitrogen, oxygen or sulfur atoms.

In particular embodiments, the reporter group is selected such that it does not substantially sub-fragment under conditions typical for the analysis of the polypeptide such as electrophoresis or chromatography. In other particular embodiments, the reporter group is chosen so that it does not substantially sub-fragment under conditions of dissociative energy applied to cause fragmentation of both bonds X' and Y' of at least a portion of selected ions of a labelled polypeptide in a mass spectrometer. Optionally, the reporter group is chosen such that eventual fragments of the reporter group are difficult or impossible to detect above background noise using MS analysis. In particular embodiments, the mass of a reporter group is intentionally selected to be different compared with the mass of the polypeptide sought to be determined or any of the expected fragments of the polypeptide. For example, the reporter's mass is chosen to be different compared to any naturally occurring amino acid or peptide, or expected fragments thereof. This facilitates polypeptide determination since, depending on the polypeptide, the lack of any possible components of the sample having the same coincident mass adds confidence to the result of any analysis.

In particular embodiments of the labelling reagents of the present invention, the reporter group of the isobaric label component is a small molecule that is non-polymeric. In further particular embodiments, the mass of a reporter is less than 250 Daltons. Such a small molecule is easily determined in the second mass analysis, free from other components of the sample having the same coincident mass in the first mass analysis. In this context, the second mass analysis is performed typically in a tandem mass spectrometer, on selected ions that are determined in the first mass analysis. Because ions of a particular mass to charge ratio are specifically selected out of the first mass analysis for possible fragmentation and further mass analysis, the non-selected ions from the first mass analysis are not carried forward to the second mass analysis and therefore do not contaminate the spectrum of the second mass analysis. Furthermore, the sensitivity of a mass spectrometer and the linearity of the detector (for purposes of quantitation) is quite robust in this low mass range. Additionally, the present state of mass spectrometer technology allows for baseline mass resolution of less than one Dalton in this mass range.

In the isobaric label components of the labelling reagents of the present invention, the balance group (BG) links the reporter group to either the isotopic label component or to a protein reactive group. In particular embodiments the balance group is selected to produce a neutral species when both bonds X' and Y' are fragmented (i.e. undergoes neutral loss upon fragmentation of both bonds X' and Y'). In particular embodiments, the balance group is a very small moiety such as a carbonyl or thiocarbonyl group. For example, the balance group comprises at least one heavy atom isotope and comprises the formula -CH₂-CO-CH₂-, CH₂-CS-CH₂-, CH₂-CNH-CH₂- or CH₂-CHR¹-CH₂-, wherein R¹ is the same or different and is an alkyl group comprising one to eight carbon atoms which optionally contain a heteroatom or a substituted or unsubstituted aryl group wherein the carbon atoms of the alkyl and aryl groups independently comprise linked hydrogen, deuterium and/or fluorine atoms. In particular embodiments, the balance group is a larger moiety. For example, the balance group is a polymer or a biopolymer. In particular embodiments, the balance group is designed to sub-fragment when subjected to dissociative energy levels, including sub-fragmentation to thereby produce only neutral fragments of the balance group.

Typically, the balance group of the isobaric label components in the labelling reagents of the present invention comprises one or more heavy atom isotopes such that its mass compensates for the difference in mass between the reporters groups, so as to obtain an identical mass for each isobaric label component of the set. The aggregate mass (i.e. the mass taken as a whole) of the reporter/balance group combination is the same for each isobaric label component and thus the mass of identical polypeptides or peptides, originating from different samples and labelled with the different labelling reagents of a set according to the present invention comprising the isobaric label components will be the same.

Identical peptides or polypeptides labelled with labelling reagents of the same set comprising the same isotopic label component but different isobaric label components are isobars, i.e. have the same weight. Thus, if one selects, from an initial mass analysis of a pooled sample mixture, an ion of a particular mass to charge ratio in the mass spectrometer, this selected ion contains the identical polypeptides from those samples in the pooled sample mixture that were labelled with the labelling reagents comprising the same isotopic label, in a proportion which corresponds to their respective concentration and/or quantity in the sample mixture.

Because the balance group acts as a mass balance for the reporter in the isobaric label components, such that the aggregate mass of the reporter group/balance group combination is the same for all labelling reagents of a set or kit, the greater the number of atoms in the balance (and reporter) group, the greater the possible number of different isomeric/isobaric label components that can be generated, i.e. the greater the number of combinations with the isotopic label component that can be obtained, resulting in an increased number of labelling reagents within a set and/or kit. Generally the reporter group is not a limiting factor and the greater the number of atoms that a balance group comprises, the greater number of potential reporter/balance group combinations exist, since isotopes can be substituted at most any position in the balance group to thereby produce a set of isomers or isobars of the balance group portion wherein the balance group portion is used to offset the differing masses of the reporter portion and thereby create a set of reporter/balance group isomers or isobars. Such diverse sets of labelling reagents components are particularly well suited for multiplex analysis of polypeptides in the same and/or different samples.

The total number of different isobaric label components that can be used to generate combinations of isotopic/isobaric labelling reagents according to the present invention is two, three, four, five, six, seven, eight, nine, ten or more. As the double labelling reagents of the present invention comprise differing combinations of the isobaric and isotopic (or allobaric) label components, the total number of labelling reagents in a set will be determined by the product of the number of different label components within a set. The number of isotopic label components are usually limited, and typically 2, thus resulting in a total number of double labelling reagents within a set of double the number of isobaric label components. The diversity of isobaric label components is determined by the number of atoms of the reporter and balance group moieties, the heavy atom isotopes available to substitute for the light isotopes and the various synthetic configurations in which the isotopes can be synthesized. As mentioned, numerous isotopically enriched basic starting materials are readily available from manufacturers such as Cambridge Isotope Laboratories and Isotec. Such isotopically enriched basic starting materials are used in the synthetic processes used to produce sets of isobaric and isomeric label components or to produce the isotopically enriched starting materials that are used in the synthetic processes used to produce sets of isobaric and isomeric label components. The preparation of isobaric label components according to the present invention at least in part corresponds to the preparation of isobaric labelling reagents disclosed in the examples of WO2004070352.

In the combined isobaric/isotopic labelling reagents of the present invention, the reporter group and balance group of the isobaric label component are connected to each other and optionally to the isotopic label component and/or protein/peptide reactive group, by bonds referred to as X' and Y', wherein X' is a bond between an atom of the reporter group and an atom of the balance group and Y' is a bond between an atom of the balance group and an atom of the isotopic label component and/or the protein/peptide reactive group of the labelling reagent. In particular embodiments bonds X' and Y' of the various isobaric labelling reagents are dissociated, when subjected to dissociative energy levels. Therefore, in particular embodiments of the methods of the invention, the dissociative energy level is adjusted in a mass spectrometer so that both bonds X' and Y' are dissociated in at least a portion of the selected ions of the isobarically labelled polypeptides or peptides . Where the reporter group is positioned terminally on the labelling reagent, fragmentation of bond X' releases the reporter group from the polypeptide so that the reporter is detectable independently from the peptide or polypeptide. Where the balance group is connected to the polypeptide or peptide through bond Y', fragmentation of bond Y' releases the reporter group/balance group combination from the polypeptide or peptide, or the balance group from the polypeptide, depending on whether or not bond X' has already been fragmented.

The reporter group of an isobaric label is generally present as the terminal part of the double labelling reagents of the present invention. In this case only one bond (X') between the reporter and the balance group needs to be disrupted to release the reporter. However in particular embodiments, the reporter group is an internal molecule in the double labelling reagents of the invention, whereby the flanking bonds are such that they allow the release of the reporter group from the labelling reagent after CID.

In the same way the distribution of the isobaric label component in particular embodiments of the double labelling reagents of the present invention is envisaged to correspond either to the classical design of isobaric labelling reagents, whereby the reporter group and the balance group are positioned adjacent to each other. Alternative embodiments of the double labelling regents of present invention comprise a reporter and a balance group which are separated from each other by other parts of the double labelling reagent, e.g. by the isotopic label component. In all of the embodiments however, the mass of the balance group compensates the mass of the reporter group.

In particular embodiments bond Y' is more labile than bond X'. In other particular embodiments bond X' can be more labile than bond Y'. In yet other particular embodiments, bonds X' and Y' have the same relative lability. In particular embodiments, the relative lability of bonds X' and Y' is adjusted with regard to an amide (peptide) bond. Bond X', bond Y' or both bonds X' and Y' are in such case more, equally labile or less labile compared to a typical amide (peptide) bond. For example, under conditions of dissociative energy, bond X' and/or bond Y' are less prone to fragmentation as compared with the peptide bond of a Z-Pro dimer or Z-Asp dimer, wherein Z is any natural amino acid and Pro and Asp are respectively Proline and Aspartic acid. In some embodiments, bonds X' and Y' will fragment with approximately the same level of dissociative energy as a typical amide bond. In some embodiments, bonds X' and Y' will fragment with a greater level of dissociative energy as compared with a typical amide bond.

In other particular embodiments, bonds X' and Y' are chosen such that fragmentation of bond Y' induces the fragmentation of bond X', and vice versa. In this way, both bonds X' and Y' fragment essentially simultaneously such that no substantial amount of polypeptide or peptide, or daughter fragment ion thereof, comprises a partial label in the second mass analysis. Substantial amount of polypeptide refers to less than 25 %, and even less than 10%, partially labelled polypeptide that is determined in the MS/MS spectrum. Because there can be a clear demarcation between labelled and unlabelled fragments of the polypeptide in the spectra of the second mass analysis (MS/MS), this feature simplifies the identification of the polypeptides based on computer assisted analysis of the daughter fragment ion spectra. Moreover, because the fragment ions of polypeptides are, in some embodiments, either fully labelled or unlabelled (but not partially labelled) with the reporter/balance group moiety, there is little or no scatter in the masses of the daughter fragment ions caused by isotopic distribution across fractured bonds such as would be the case where isotopes were present on each side of a single labile bond of a partially labelled polypeptide routinely determined in the second mass analysis.

The reaction between the labelling reagents of the present invention and the protein or peptide to be labelled is ensured by the presence of a protein/peptide reactive group. The protein/peptide reactive group (PRG) on the labelling reagent is a group that selectively reacts with certain protein or peptide functional groups or is a substrate of an enzyme of interest. Examples of suitable protein/peptide reactive groups and the functional groups they react with are provided below:
- Thiol-reactive groups react with the side chain of cysteine. Thiol-reactive groups include epoxides, alpha-haloacyl group, nitriles, sulfonated alkyl or aryl thiols, alkyl halides, aryl amides and maleimides. A particular example is iodoacetamide or a derivative thereof.
- Amino-reactive groups react with the epsilon amine group of the side chain of lysine or react with the amine at the N-terminus of a polypeptide. Amino reactive groups tag amino groups in proteins and include sulfonyl halides, isocyanates, isothiocyanantes, active esters, including tetrafluorophenyl esters, pentafluorophenyl esters, N-hydroxysuccinimidyl esters, N-hydroxysulfosuccinimidyl esters, 2-nitrophenyl esters, 4-nitrophenyl esters, 2,4-dinitrophenylesters and 2,4-dihalophenyl esters, acid halides, and acid anyhydrides and mixed anhydrides. In addition, amino reactive groups include aldehydes or ketones in the presence or absence of NaBH₄ or NaCNBH₃.
- Carboxylic acid-reactive groups react with the side chains of aspartic acid and glutamic acid or react with the C-terminus of a polypeptide. Carboxylic acid reactive groups include amines or alcohols in the presence of a coupling agent such as dicyclohexylcarbodiimide, or 2,3,5,6-tetrafluorophenyl trifluoroacetate and in the presence or absence of a coupling catalyst such as 4-dimethylaminopyridine; and transition metal-diamine complexes including Cu(II)phenanthroline.
- Imidazoles reactive groups react with histidine.
- Ester reactive groups include amines which, for example, react with homoserine lactone. Methionine is converted upon homoserine lactone during CNBr cleavage.
- Phosphate reactive groups react with phosphorylated amino acids such as phosphoSer, PhosphoThr and PhosphoTyr. Phosphate reactive groups include chelated metal where the metal is, for example Fe(III) or Ga(III), chelated to, for example, nitrilotriacetiac acid or iminodiacetic acid. These agents react with posphorylated amino acids (such as phosphoserine, phosphothreonine, and phosphotyrosine).
- Aldehyde or ketone reactive groups include amine plus NaBH₄ or NaCNBH₃, or these reagents after first treating a carbohydrate with periodate to generate an aldehyde or ketone.
- Hydroxyl reactive groups react with serine and threonine. Hydroxyl reactive groups include trityl-halides or a silyl-halide reactive moiety which is either substituted or unsubstituted.

The double labels of the present invention are bound to a protein or peptide by interaction between the protein/peptide reactive group of the labelling reagent and a functional group present within the protein or peptide. This functional group can be pre-existing in the polypeptide as present in the sample or can be created thereon, optionally after the isolation of the polypeptide. For example, a carboxylic acid group can be modified with water-soluble carbodiimide (e.g. 1-(3-dimethylaminopropyl)-3-ethylcarbodiimide hydrochloride; EDC) to thereby obtain an electrophilic group that reacts with a nucleophile such as an amine group. Activation of the carboxylic acid group of a labeling reagent with EDC can be performed in the presence of an amine. The use of carbodiimides in combination with NH₂-CH₂-CH₂-NH₂ allows the conversion of carboxylgroups into amine groups. The use of EDC in combination with NH₂-CH₂-CH₂-SH together ensures the conversion of carboxylgroups into thiol groups.

A non-limiting list of other compounds which are suitable to modify or to introduce functional groups on a polypeptide includes 2-Aminoethyl-2'-aminoethanethiol-sulfonate which is a thiol-reactive amination reagent that converts a thiol group into a reducing agent cleavable amine group; aminoethyl-8 reagent that converts free thiols into primary amine groups; BMPA that modifies thiol groups to carboxyl groups; MSA which is amine-reactive with latent carboxyl group (it converts amines to protected carboxyls. Carboxyls are unmasked at pH 9.5 in phosphate buffer); SATA which reacts with primary amines to add protected thiols; Hydroxylamine HCl which is a highly effective reagent for deblocking SATA and SATP modified proteins to generate a free thiol and Traut's Reagent which is a water-soluble reagent that reacts with primary amines at pH 7-10 to introduce thiol groups. Such reagents available from e.g. Pierce Chemicals (IL, USA).

It is also envisaged that one or more functional groups are created in a peptide or polypeptide by removal of a protecting group. Consequently, in particular embodiments, functional groups in a polypeptide are changed or added. In particular embodiments the added functional groups are groups which do not occur naturally in proteins.

In other embodiments of the double labelling reagents suitable in the context of the present invention, the PRG groups are in fact substrates for a particular protein-modifying enzyme. This enzyme can be an enzyme which is involved, in vivo, in post translation modifications. Some of these enzymes are associated with a disease state or birth defect. Examples hereof are acid phosphatase, alkaline phosphatase, alanine aminotransferase, amylase, angiotensin converting enzyme, aspartate aminotransferase, creatine kinase, gamma-glutamyltransferase, lipase, lactate dehydrogenase, and glucose-6-phosphate dehydrogenase, proteases, and esterases.

According to particular embodiments of the labelling reagents of the invention, the isotopic label component and the isobaric label component make up different parts of the labelling reagent, separated by a bond. Suitable isotopic and isobaric label components according to these embodiments are described above.

However, to simplify the manufacture of the reagent, in particular the incorporation of isotopes, the isotope of the isotopic label component can be incorporated in/on another part of the labelling reagent, more particularly in/on the isobaric label component, either in the reporter group or in the balance group thereof.

According to a particular embodiment, the balance group of the isobaric groups has the structure -C-CO-C- (see Figure 3)(such as for the commercially available isobaric labels), or a larger group, such as those described above, for example -CH₂-CHR¹-CH₂-, wherein R¹ is the same or different and is an alkyl group comprising one to eight carbon atoms, which comprises the isotopic label component which is optionally a heteroatom or a substituted or unsubstituted aryl group wherein the carbon atoms of the alkyl and aryl groups independently comprise linked hydrogen, deuterium and/or fluorine atoms. In order for the balance group of the isobaric label component to function also as the isotopic label component, such -CH₂-CHR¹-CH₂- group on the one hand carries the required number of isotopes to provide the balance with the reporter group and on the other hand contains the required number of isotopes replacements to generate a mass difference between the different labelling reagents in one set.

Alternatively the mass difference between individual labelling reagents is obtained by changing one or more atoms in a labelling reagent by other atoms thereby ensuring that the chemical structure and the physicochemical features of the labelling reagent are minimally changed to ensure the essentially identical behaviour of differentially labelled identical peptides prior to MS, i.e. during electrophoresis or chromatography, regardless of the attached label.

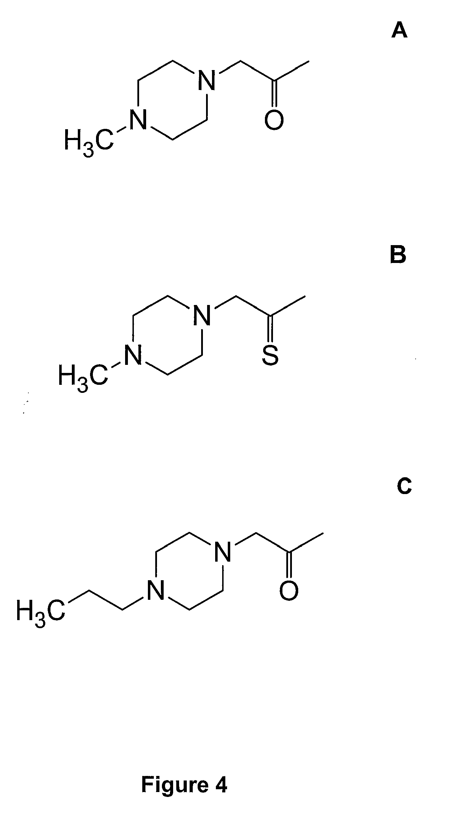

For example, a set of 8 labelling reagents is envisaged wherein one set of 4 labelling reagents have an isobaric label component, comprising an oxygen as depicted in Figure 3. In the other set of 4 labelling reagents, the oxygen atom in the isobaric label component is replaced by sulphur (see figure 4 and balancing is obtained in the same way as shown in figure 3 by replacing ³²S by ³⁴S. The consequences of this modification on the mass of the isobaric label components is illustrated in Table 2.

**Table 2 : Generation of mass difference by modification of the structure the balance group of an isobaric label.**

| Label | 1 | 2 | 3 | 4 | 5 | 6 | 7 | 8 |
|---|---|---|---|---|---|---|---|---|
| Mass reporter group | 114 | 115 | 116 | 117 | 114 | 115 | 116 | 117 |
| | Balance group -CO- | | | | balance group -CS- | | | |
| Mass balance group | 31 | 30 | 29 | 28 | 51 | 50 | 49 | 48 |
| | | | | | | | | |
| Sum | 145 | 145 | 145 | 145 | 165 | 165 | 165 | 165 |

The slight chemical difference between the double-labelled peptides which is obtained by replacing a single carbon in the label by sulphur will change the behaviour of such peptide on separation systems such as liquid chromatography only to a limited extent. The difference between the mass of carbon and sulphur allows the separation of two sets of labelled peptides based on their mass. For each of these two sets, the different reporters groups will be generated in MS/MS as usual.

In another embodiment of the present invention the mass difference between differential labelling reagents is obtained by incorporating additional isotopes in the reporter group of the isobaric label component. In the isobaric label component as depicted in Figure 3, an additional deuterium atom can be incorporated in the methyl-piperazine group, to generate an isotopic label of the label of Figure 3. However, when higher multiplexing is envisaged using sets of labelling reagents with 3 or more different masses, the methyl piperazine group becomes too small to harbour all these additional isotopes. Thus, as an example, labelling reagents are provided with a propyl piperazine group (see figure 4C) wherein different deuterium and ¹³C isotopes are incorporated.

Table 3 shows the masses of the reporter and balance groups for a set of 8 labelling reagents with a propyl piperazine group, wherein the mass difference is obtained by incorporating two ¹³C and two ²H isotopes in the propyl side chain.

**Table 3: Generation of mass difference by modification of the reporter group of an isobaric label.**

| label | 1 | 2 | 3 | 4 | 5 | 6 | 7 | 8 |
|---|---|---|---|---|---|---|---|---|
| mass reporter group | 142 | 143 | 144 | 145 | 146 | 147 | 148 | 149 |
| | without isotopes in propyl side chain | | | | with two 13C and two deuterium atoms in propyl side chain | | | |
| mass balance group | 31 | 30 | 29 | 28 | 31 | 30 | 29 | 28 |
| | | | | | | | | |
| sum | 173 | 173 | 173 | 173 | 177 | 177 | 177 | 177 |

This design of the labels, as present in Table 3 has the advantage that the mass of a reporter group is unique for each of the labelled peptides in a pooled sample, and serves as an internal control.

Similarly, as explained above for the balance group, the difference in mass between individual labelling reagents can also be obtained by replacing an atom in the reporter group by another atom provided that the change in structure and physicochemical properties is minimal.

Summarising, by providing the isotopic label component within the reporter group and/or the balance group of a conventional isobaric label component, there is no need to design a separate part in a labelling reagent for the incorporation of an isotope.

In particular embodiments, the double labelling reagents of the present invention comprise an affinity tag (A), which allows to selectively isolate those polypeptides that have reacted with the labelling reagent, i.e. have been labelled. While the affinity tag, in principle can be present at any position on the reagent, it is typically placed at a position where it does not interfere with the release of reporter group after CID. In particular embodiments, the affinity tag is located on a side chain of the isotopic label component. Although affinity tags can be bulky, they can be bound to the labelling reagent with a cleavable group. Generally affinity tags are removed from the label on the polypeptide prior to detection, leaving only a scar on the label.

As indicated above, the presence of an affinity tag in the double labelling reagents of the present invention, allow the selective binding of labelled peptides or polypeptides, either covalently or non-covalently and with high affinity to a capture reagent (CR). Typically the binding of the affinity tag to the capture reagent is strong such that it is resistant to extensive and/or multiple washing with any or a combination of a variety of solutions which ensure the removal of peptides or polypeptides non-specifically bound to the capture reagent. Typically the affinity tag does not undergo peptide-like fragmentation during MS analysis.

The removal of peptides or polypeptides bound to the CR through an affinity tag can be ensured by addition of a displacing ligand (DL), described below, or by changing the temperature or solvent conditions.

According to a particular embodiment, it is envisaged that, in order to further reduce the complexity of the sample for analysis, an extraction of the double labelled proteins or peptides is envisaged in the context of the methods and tools of the present invention. For use in the context of these embodiments, the nature of the affinity tags is not critical, as long as it allows the selective binding to a capture reagent (CR) and optionally removal therefrom, without affecting the peptide or polypeptide bound to the affinity tag.

Accordingly, non-limiting examples of Affinity tags (A) and capture reagent (CR) pairs include:
- d-biotin or structurally modified biotin-based reagents, including d-iminobiotin, which bind to avidin/streptavidin (for example as strepavidin-Agarose, oligomeric-avidin-Agarose, or monomeric-avidin-Agarose)
- a 1,2-diol, such as 1,2-dihydroxyethane (HO-CH₂ -CH₂ -OH), and other 1,2-dihydroxyalkanes including those of cyclic alkanes, e.g., 1,2-dihydroxycyclohexane which bind to an alkyl or aryl boronic acid or boronic acid esters, such as phenyl-B(OH)₂ or hexyl-B(OEthyl)₂ (eg attached via an alkyl or aryl group to a support such as agarose)
- maltose which binds to Maltose Binding Protein; or other sugar/Sugar Binding Protein pairs, or more generally to any ligand/Ligand Binding Protein pair which obeys to the above mentioned criteria of affinity tags.
- a hapten, such as dinitrophenyl group, which binds to the corresponding anti-hapten antibody such as anti-dinitrophenyl-IgG;
- a ligand which binds to a transition metal, for example, an oligomeric histidine (so called 6His-tag) will bind to Ni(II), the transition metal CR is in particular embodiments used in the form of a resin-bound chelated transition metal, such as nitrilotriacetic acid-chelated Ni(II) or iminodiacetic acid-chelated Ni(II);
- glutathione which binds to glutathione-S-transferase.

In particular embodiments of the present invention, an affinity purification of double labelled peptides or polypeptides is performed prior to analysis by MS. Accordingly, as the bound peptides or polypeptides are required for further analysis, removal from the capture reagent or dissociation of the affinity tag from the peptide is required. This can be ensured in different ways.

In particular embodiments, the affinity tag is cleavable from the labelling reagent, leaving the isobaric and allobaric (isotopic) label components of the reagent intact. As indicated above, the location of the affinity tag on the labelling reagents of the present invention is not critical. Thus, the affinity tag can be bound to either the isotopic or the isobaric label component in the labelling reagent by way of a chemically or enzymatically cleavable bond.

In particular embodiments, the affinity tag is connected to the double labelling reagent by a cleavable bond (such as, but not limited to an acid labile, thiol labile, base labile, periodate labile, or hydroxylamine labile bond).

In further particular embodiments, the bond between affinity tag and labelling reagent can also be cleavable by chemical, thermal or photochemical reaction. A suitbale photocleavable groups is 1-(2-nitrophenyl)-ethyl. Thermally labile bonds are for example, double-stranded nucleic acids, double strands of a nucleic acid with peptide nucleic acid, or double stranded peptide nucleic acid strands which will dissociate upon heating. Cleavable groups also include those having disulfide bonds, acid or base labile groups, including among others, diarylmethyl or trimethylarylmethyl groups, silyl ethers, carbamates, oxyesters, thiesters, thionoesters, and alpha-fluorinated amides and esters. Enzymatically cleavable groups are for example, protease-sensitive amides or esters, beta-lactamase-sensitive betalactam analogues and bonds that are nuclease-cleavable, or glycosidase-cleavable.

According to this embodiment, the affinity tag can be removed by treatment of the labelled protein or peptide with a suitable reagent. Removal of the affinity tag may also be desirable for other reasons, e.g. to avoid interference in analysis of the peptide, e.g. in MS. Typically, the affinity tag is cleaved off after the affinity isolation of labelled peptides or polypeptides.

Alternatively, the affinity tag can be bound to the labelling reagents of the present invention by a non-cleavable bond. In order to ensure that the affinity purified peptides can be recuperated, an affinity tag is used which can be dissociated from the capture reagent. In particular embodiments, biotin and biotin-based affinity tags are used. Of particular interest are structurally modified biotins, such as d-iminobiotin, which will elute from avidin or streptavidin columns under solvent conditions compatible with ESI-MS analysis, such as dilute acids containing 10-20% organic solvent. It has been established that d-iminobiotin tagged compounds will elute in solvents below pH 4.

Additionally or alternatively, displacement ligands (DL) are used to displace the affinity tag (A) (and the peptide or protein bound thereto) from the capture reagent (CR). When eluting with a DL at least a fraction of this DL will be present in the eluent comprising the peptide(s) of interest. In particular embodiments the methods of the invention can comprise the use of a DL which is a molecule which does not undergo peptide-like fragmentation during MS analysis, and of which the presence in a sample does not significantly suppress the ionization of the tagged peptide, substrate or reaction product conjugates. Particularly, the displacement ligand can be chosen such that it itself is minimally ionized during mass spectrometric analysis and that the formation of ions composed of DL clusters is minimal.

The nature of a suitable displacement ligand (DL), depends upon the A and CR that are employed. In general, the DL is selected to displace A from CR in a reasonable time scale, at most within a week of its addition, but more preferably within a few minutes or up to an hour. The affinity of DL for CR should be comparable or stronger than the affinity of the tagged compounds containing A for CR. Furthermore, the DL should be soluble in the solvent used during the elution of tagged compounds containing the affinity tag A, from CR. In particular embodiments the DL corresponds to a free affinity tag A or a derivative or structural modification of A. Examples of displacement ligands thus include, d-biotin or d-biotin derivatives.

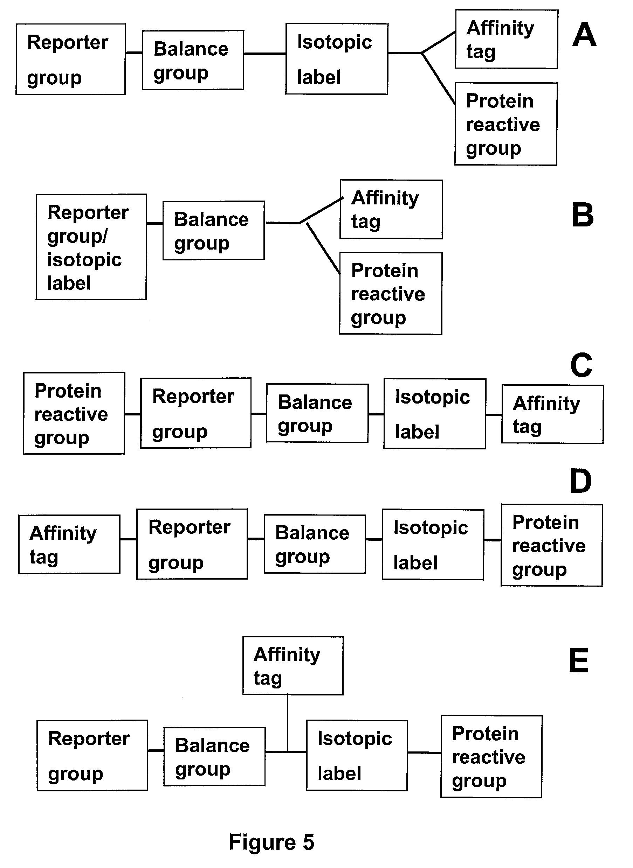

The isotopic (IT) and isobaric (IB) label components, protein reactive group (PRG) and optional affinity tags (A) which make up the parts of the labelling reagents of the present invention can be arranged in different possible configurations. A non-limiting list of possible combinations is shown in Figure 5. Requirements herein are that the PRG of the labelling reagent can react with a functional group on a polypeptide and that the reporter group of the isobaric label component can be released upon CID.

The bonds between different parts, other than those flanking the reporter group can be connected to each other via any suitable chemistry available to the skilled person. For example the chemical structure of the isotopic label component mentioned above, or the groups B1 or B2 as indicated therein can be used to connect the different parts of the labelling reagent.

In another aspect of the present invention methods and assays are provided, wherein samples are differentially labelled using the double labelling reagents of the present invention, to allow simultaneous analysis with MS.

Accordingly, the present invention provides methods for simultaneously analysing the presence of one or more polypeptides comprising a functional group in different samples. The methods of the invention comprise a labelling step wherein each sample is labelled with one of a set of differential double labelling reagents, by allowing the labelling reagents to react with a functional group of the one or more polypeptides in the samples, or peptides generated therefrom. As describe in detail above, the set differential double labelling reagents have the same or essentially the same chemical structure and carry an isotopic and an isobaric label component. Each labelling reagent comprises a unique combination of an isotopic and an isobaric label component.

In the steps following the labelling, the labelled peptides are isolated and analysed for each of the samples.

Ideally, in the analysis methods envisaged in the context of the present invention, the labelling step targets almost every protein in the sample to obtain a maximal coverage of the proteome in a sample.

At the same time, when considering each individual protein, the number of functional groups in that protein is ideally low, so as to reduce the complexity of the analysis (number of peptides to be analysed from this protein in a sample is only one or a limited number).

Cysteine is often used as functional group for labelling because cysteine labelling provides a compromise between maximal coverage (labelling every protein in a sample) and minimal complexity (labelling every protein in a sample only once or a limited number of times). Cysteine is present in about 85 % of all proteins in Genbank and on average occurs with a frequency of 2 % (one in each 50 amino acids) in a polypeptide sequence. Furthermore, labelling with cysteine reactive reagents does not modify other parts of a protein as is the case with the labelling amine groups which occur on both Lysine and the aminoterminus or as is the case with labelling carboxyl groups which occur on both Aspartic acid, Glutamic acid and the carboxyterminus. According to one embodiment of the methods of the invention, the labelling is thus performed through a cysteine functional group. In a particular embodiment, prior to the labelling step, the samples are reduced to ensure the presence of an accessible thiol functional group on the cysteines in the proteins of the sample.

Moreover, the chance that cysteine appears in either the C-terminal peptide or the N-terminal peptide of a given enzymatic digest decreases with the number of recognition sites for such enzyme in a protein. This number normally increases proportionally with the length of a polypeptide. Thus labelling methods wherein cysteine is used as the functional group of the labelling reagent are very well suited for determining relative expression levels of a protein in a sample.

According to a particular embodiment, as indicated above, the methods of the invention comprise a cleaving step of proteins into peptides prior to or after the labelling step. This is generally performed to allow the analysis of smaller peptides, rather than the full-length proteins. It will be easier to separate peptides than proteins on high throughput systems such as LC, and to interpret sequence data from peptides in MS/MS.

Suitable chemicals for protein cleavage include cyanogen bromide, BNPS skatole (2-(2'-Nitrophenylsulfonyl)-3-methyl-3-Bromoindolenine), formic acid, hydroxylamine, iodobenzoic acid, NTCB + Ni (2 nitro 5 thiocyanobenzoid acid). Suitable proteolytic enzymes include Asp-N Endopeptidase, Caspase 1, 2, 3, 4, 5, 6, 7, 8, 9, or 10, Chymotrypsin; Clostripain, Enterokinase, Factor Xa, Glutamyl Endopeptidase, Granzyme B, LysC Lysyl endopeptidase, Papain, Pepsin, Proline-Endopeptidase, Proteinase K, Staphylococal peptidase I, Thermolysin, Thrombin, Trypsin.

Depending on the type of protein sample a combination of chemical and enzymatic cleavage is performed or a double enzymatic digestion is performed.

Despite the variety in enzymes and chemicals, trypsin still remains the enzyme with the highest specificity (Lysine and Arginine) and efficiency. In vertebrates Lysine occurs in proteins with a frequency of 7,4 % and Arginine with a frequency of 4,2 %. A tryptic digest results thus in peptides with an average length of 9 amino acids on non-modified proteins and of 25 amino acids when lysine is modified to an extent where it is no substrate anymore for trypsin. Such peptides are well suited for chromatographic and MS techniques.

According to a particular embodiment of the methods of the invention, the cleavage of proteins is performed after the labelling. This allows to reduce the complexity of a sample by isolating only those peptides which carry a label on a functional group.

According to a particular embodiment labelled proteins are digested with trypsin. According to a more particular embodiment labelled proteins are treated with an acetylating agent to modify the side chains of lysine before a tryptic digestion is performed. As a consequence the aminoterminus of a polypeptide will be acetylated equally.

According to another particular embodiment, labelled proteins are digested with a lysine specific protease. As a results all peptides obtained from such a digest will have an amine group at their aminoterminus and at the side chain of the carboxyterminal lysine (with the exception of the carboxyterminal peptide which may have another carboxyterminal amino acid).

Proteins which are double labelled in the labelling step but which do not comprise a protein cleavage site can participate as such in the remainder of the procedure as cleaved peptides.

According to particular embodiments of the methods of the present invention, the labelling reagents used comprise an affinity tag, and the methods comprise the step of double labelling the proteins present in the samples, cleaving the double labelled proteins and isolating the double-labelled peptides based on the affinity tag present in the label on the peptides. This affinity isolation allows for a reduction of the complexity of the sample for further analysis. When a protein cleavage is performed, those peptides which did not comprise a functional group and thus were not labelled, are removed.

The methods of the present invention, comprise a step of pooling two or more samples which are differentially labelled in the first and second labelling step. By pooling of the different samples a polypeptide sample mix is obtained. This allows the immediate comparison of the different samples in the analysis.

Where the protein samples used in the methods of the invention are complex, the pooled sample will be subjected to one or more peptide separation techniques to selectively isolate double labelled polypeptides or peptides with a same amino acid sequence from the polypeptide sample mix.

According to the present invention, the chemical structure of the combined isobaric/isotopic labels is the same or is essentially the same within one set of labelling reagents, such that it will not generate a significant difference in properties in (multi-dimensional) chromatography techniques, between identical peptides that are differently labelled.

Suitable separation techniques, which allow the separation of a complex protein or peptide sample into two or more, up to multiple fractions (in the context of the isolation of double-labelled peptides) are known to the skilled person and include, but are not limited to isoelectric focusing, SDS PAGE, 2-dimensional gel electrophoresis, size-exclusion chromatography, ion exchange chromatography, reversed-phase HPLC, affinity chromatography, ... etc.

When the sample consists of larger peptides (e.g. between Mr 3000 and 100.000) 2D GE can be used. For peptide samples, obtained from e.g. proteolytic digestions, 2D LC approaches are more suitable for separation, and also the automation and throughput is significantly better. Several technologies to separate protein/peptide digests by liquid chromatography have been described, reversed-phase (RP)-HPLC, and 2-dimensional liquid chromatography. Also capillary electrophoresis (CE) is a method suitable for the separation peptides. 2D-LC generally uses ion-exchange columns (usually, strong cation exchange, SCX) on-line coupled with a reversed phase column, operated in a series of cycles. In each cycle the salt concentration is increased in the ion-exchange column, in order to elute peptides according to their ionic charge into the reversed phase system. Herein the peptides are separated on hydrophobicity by e.g. a gradient with CH₃CN.

Many parameters influence the resolution power and subsequently the number of proteins that can be displayed by LC-MS. Usually, the 'on-line' configuration between the first-dimension separation technique (SCX) and the second-dimension RP-HPLC separation approach is set up for sample fractionation. Ion exchange chromatography can be performed by stepwise elution with increasing salt concentration or by a gradient of salt. Typically, SCX is performed in the presence of, e.g. up to 30% acetonitrile, to minimize hydrophobic interactions during SCX chromatography. Prior to Reversed Phase chromatography on e.g. a C18 column, organic solvents such as acetontile are removed, or strongly reduced by e.g. evaporation.

Accordingly devices suitable for performing the methods of the present invention optionally contain or are connected to one or more suitable separation instruments, such as electrophoresis instruments, chromatography instruments, such as, but not limited to capillary electrophoresis (CE) instruments, reversed-phase (RP)-HPLC instruments, and/or multi-dimensional liquid chromatography instruments,... etc.

The present invention provides tools and methods for the simultaneous identification (by MS/MS) and/or quantitation (by MS or MS/MS) of proteins in different samples. More particularly, the methods of the present invention relate to the analysing of the relative occurrence of the isolated double labelled polypeptides or peptides by Mass spectroscopy, followed by the identification of peptides.

Accordingly, the devices for performing the methods of the present invention comprise one or more mass spectrometric instruments.

Mass measurements by spectrometry are performed by the ionization of analytes into the gas phase. A typical mass spectrometric instrument consists of 3 components, an ion source to generate ions from the molecules of interest, a mass analyzer, which determines the mass-to-charge ratio (m/z) of the ionized molecules, and a detector that registers and counts the number of ions for each individual m/z value. Each feature in an MS spectrum is defined by two values, m/z and a measure on the number of ions, which reached the detector of the instrument.

The ionization of proteins or peptides for mass analysis in a spectrometer is usually performed by Electro-spray ionization (ESI) or matrix-assisted laser desorption/ionisation (MALDI).

During the ESI process analtyes are directly ionized out of solution and ESI is therefore often directly coupled to liquid-chromatographic separation tools (e.g., reversed phase HPLC). MALDI vaporizes via laser pulses dry samples mixed with small organic molecules that absorb the laser energy like cinnamic acid to make the process more effective. The mass analyser is a key component of the mass spectrometer; important parameters are sensitivity, resolution, and mass accuracy. There are five basic types of mass analysers currently used in proteomics. These include the ion trap, time-of-flight (TOF), quadrupole, Orbitrap, and Fourier transform ion cyclotron (FTICR-MS) analysers. Tandem MS or MS/MS can be performed in time (ion trap) and in place (with all hybrid instruments such as e.g. LTQ-FTICR, LTQ-Orbitrap, Q-TOF, TOF-TOF, triple quad and hybrid triple quadrupole/linear ion trap (QTRAP))

As detailed herein, the spectrum generated on mass spectrometer of a polypeptide or peptide which has been previously isolated from a pool of differentially labelled samples, contains a set of peaks with the characteristic mass differences between the different isotopic label components (the number of peaks will be less if a protein wherein this polypeptide occurs is not expressed at all in one of the samples). Each of these peaks are further analysed by tandem MS to release the reporter group of the isobaric label component for identification and further quantitation of the differently labelled peptides. Based on Mr of the polypeptide, the amino acid composition or the amino acid sequence of the individual peptides, the identity of the peptides is determined.

The methods of the present invention are hereafter illustrated by a number of labelling schemes.

As detailed above and further clarified hereafter in the different illustrative labelling schemes, the fragmentation of peptides or the use of an affinity tag is not essential for all embodiments of the present invention.

The following labelling scheme (see Figure 6) discloses an embodiment of a labelling of 8 protein samples using the double labelling reagents of the present invention, with a protein reactive group (PRG),which is a cysteine reactive group.

In a pre-processing step the samples are reduced to ensure the presence of an accessible thiol functional group on cysteine, and are acetylated to block the amines of the side chain of lysine.

The different steps of the labelling scheme comprise:
- The 8 protein samples are each labelled with one of a set of double labelling reagents, wherein each labelling reagent has a unique combination of an isotopic and an isobaric label, and the labelling reagents are biotin tagged and comprise a cysteine reactive group.
- After the labelling, already at this stage all samples are pooled and treated as one reaction mixture, which decreases variations due to the manipulation of individual samples.
- The cysteine labelled samples so obtained are digested with trypsin.
- To reduce the number of peptides for further analysis, only the double labelled peptides are isolated via the biotin affinity tag on the label using (strept)avidin affinity techniques.

After the affinity step each peptide now has a label with an isotopic and an isobaric label component. Consequently, every peptide is informative upon analysis via MS. The method further includes the steps wherein:
- The pooled peptides are separated by e.g. liquid chromatography. Herein peptides that are identical but which originate from a different sample and are thus differently labelled will behave in an essentially identical manner. Despite the different combinations of isotopic and isobaric label component which is present on each of the peptides from a different sample, this will not affect their elution as the different labels in each label set have the same chemical formula or essentially the same formula.
- Each separated peptide fraction is analysed by MS, which should generate two signals, with a different mass, corresponding to the presence of a "light" or a "heavy" isotopic label component. Hereafter, each peptide with a different isotope is further fragmented whereby the reporter group is released from the isobaric label component present on each of the peptides. The relative amount of reporter indicates the relative amount of peptide which was labelled with that reporter. MS data are further used to determine the sequence of the peptide and to identify the protein it is derived from. A theoretical example of determining the relative amount of each labelled peptide in a peptide mixture is shown in example 1 herein.
   The method of the invention as illustrated above, using a set of labelling reagents with combinations of 2 different isotopic label components and 4 different isobaric label components, allows multiplexing of different protein samples up to a complexity of 8. When additional isobaric and/or isotopic label components are used, this allows for increased multiplexing. The multiplexicity is determined by the product of the number of different isotopic label components times the number of different isobaric label components.
   The above described exemplary labelling protocol is suitable for determining e.g. differences in expression level in-between individual proteins in different samples using internal peptides. Different alternative protocols are envisaged, depending on particular applications.
   The most conventional method as outlined above uses a labelling on a side chain of a peptide. Typically the labelling on the side chain is performed on cysteine. However as mentioned above about 15 % of proteins in the protein database have no cysteine and will not be labelled. The chance that no cysteine occurs in a protein increases with decreasing protein length. This will be equally true for the labelling at side chains of amino acids other than cysteine. The shorter the protein, the smaller the chance for labelling on the side chain of an amino acid.
   To study low Mr proteins, the double labelling reagents and methods of the present invention whereby protein/peptide reactive groups interacting with functional groups which are primary amines or carboxyl groups are of particular interest. Indeed, these groups always occur in each protein at respectively the aminoterminus and the carboxyterminus. According to a particular embodiment, the methods of the invention comprise the step of fractionating the samples in one or more low Mr fractions using gel filtration chromatography or size exclusion methods (dialysis or ultrafiltration). Thereafter the low Mr protein fraction of the samples are labelled without further protein cleavage, as the limited size of such protein samples makes them directly suitable for liquid chromatography techniques. In this embodiment of the methods of the invention, it is not necessary to use affinity tags, because every protein in the low Mr fraction is labelled.
   In another embodiment, the reagents of the present method are used to specifically label the N-terminal or the C-terminal peptide of a protein.
   For the isolation of N-terminal peptides, the amine groups on a protein (Lysine and the N-terminus) are first modified (e.g. alkylated or acetylated). The side chain of Asp and Glu is also modified. Thereafter the protein is cleaved (e.g. with trypsin) whereby all peptides, apart from the N-terminal peptide obtain a novel accessible reactive amine group. This group is reacted with a protein reactive affinity tag (e.g. NHS-biotin). Hereafter the internal and C-terminal peptides are isolated using affinity chromatography. The remaining N-terminal peptide is now reacted on its C-terminus with a labelling reagent with a carboxyl reactive group. For this procedure there is no need for an affinity tag on the labelling reagent, as the peptides of interest have indeed been affinity-purified previously already. The use of a tag on the labelling reagent and the subsequent affinity purification can still be envisaged to ensure that only properly labelled peptides are subjected to further analysis. In this method, the modification of Asp and Glu prior to the cleavage, avoids labelling of amino acid side chains, but is not essential for the procedure.
   A similar method can be performed for the isolation of C-terminal peptides, wherein carboxyl groups are modified and the peptides generated upon cleavage are reacted with an affinity tag, which carries a group that is reactive towards carboxylgroups. The labelling reagent accordingly comprises a protein/peptide reactive group, which is reactive with the novel N-terminus generated upon cleavage.
   The labelling of either the N-terminal or C-terminal peptide of a protein has certain advantages.
   In the first place, all proteins in a sample are detected, while labelling at the side chain of an amino acid will overlook those proteins wherein that particular functional will not occur. In the second place, even proteins with a blocked N-terminus will be detected. Using this method all amino termini are blocked, either in vivo, either by the labelling method
   Another exemplary labeling scheme according to the methods of the present invention is one that is considered particularly suitable e.g. to study proteolytic processing of proteins (so-called degradomics) in samples. An example of differential N-terminal processing is shown in Table 1 for a hypothetical protein with an N-terminal sequence 1-9 wherein amino acid 4 and 9 is Arginine. When the protein is not processed, all eight differentially labelled N-terminal peptides elute as one fraction after chromatography, are split up into different fractions comprising different isotopic labels in MS and each of these further generate a set of different reporter groups from the isobaric label components in MS/MS.
   In the situation as outlined below in Table 1, the labelling of the N-terminal peptides of the processed proteins leads to 4 different N-terminal peptides, which will elute a different positions on high performance chromatography systems such as reversed phase HPLC chromatography. Accordingly, such a peptide fraction can even appear as a single peak on MS when such peptides have the same isotopic label but carry different isobaric labels.

**Table 1: differential N-terminal processing**

| SAMPLE | SEQUENCE | processing | N-terminal tryptic peptide |
|---|---|---|---|
| 1 | 123(4R)5678(9R).. | none | 123(4R) |
| 2 | 123(4R)5678(9R).. | none | 123(4R) |
| 3 | 123(4R)5678(9R).. | 1 | 23(4R) |
| 4 | 123(4R)5678(9R).. | 1 | 23(4R) |
| 5 | 123(4R)5678(9R).. | 2 | 3(4R) |
| 6 | 123(4R)5678(9R).. | 2 | 3(4R) |
| 7 | 123(4R)5678(9R).. | 123(4R) | 5678(9R) |
| 8 | 123(4R)5678(9R).. | 123(4R) | 5678(9R) |

Nevertheless for every sample, regardless the extent of processing, an N-terminal peptide is determined for some part of the protein it was derived from.

In the general methods of the present invention (i.e. where the nature of the functional group is not determined by the nature of the sample or the desired result), the choice of functional group to be modified on a polypeptide can be influenced by different factors, such as
- the type of protein reactive groups present on the available labelling reagents
- the presence or absence of an affinity tag in the available labelling reagent.

The methods of the present invention are useful for the detection of biomarkers, e.g. in the context of disease. Depending on the sample, a large number of peptides are analysed by MS and MS/MS. This amount can be as high as several hundreds or even more than thousand. For many of them, the relative amount in between differentially labelled versions of the same peptide will be constant regardless of the origin of the sample. However, significant differences will be observed for a limited number of peptides which are correlated with a condition of the disease as represented by a certain sample. Analysis of these peptides themselves makes it possible to determine the protein from which the peptide originates. The analysis will be even more reliable when it is observed that, for different peptides from a same protein, an identical expression level is detected.

Another aspect of the invention relates to a device for multiplex analysis of protein samples (100) comprising at least two sample sources (101), a labelling unit (103), with corresponding label sources (104), a separation unit (107), a mass spectrometers unit (108) and a control circuitry and data analysis unit (109). In particular embodiments separation unit (107) comprises two consecutively linked separation systems (1107) and (2107), wherein the first separation system (1107) is e.g. a 2D gelelectrophoresis system or a cation exchange chromatography system and separation system, and the second separation system (2107) is typically a HPLC reversed phase system.

Mass spectrometer element 108 is an MS/MS spectrometer which separates isotopic forms and wherein upon CID, the reporter groups of the isobaric labels are differentially detected and wherein *de novo* peptide sequencing can be performed. MS/MS analysis can be done using 2 fundamentally different instruments. In the first type of instrument, the ion trap in which MS/MS analysis is done in the same iontrap where MS is performed, but MS/MS is done in time (trap is filled, all ions are ejected except ion(s) of interest and CID is performed and the fragment ions are scanned). The second type of instruments, hybrid instruments (triple quad, q-tof, ltq-ftms, ltq-orbitrap), separate MS/MS in place. e.g. parent selection is done in the first mass analyzer and fragments are scanned in the second mass analyzer.

The device can further comprise a number of optional elements such as a sample preparation unit (102) wherein e.g. sample lysis and immunodepletion takes place, a protein cleavage unit (105) and an affinity purification unit (106) to selectively isolate labelled polypeptides carrying an affinity tag (e.g. biotin - avidin affinity purification). Suitable devices for incorporation into the devices of the present invention as mass spectrometer units and separation units are described above.

It will be appreciated that the labelling methods of the present invention are applicable in proteomics, protein expression profiling, biomarker discovery and target discovery. The high multiplexing of the methods of the present invention allows to analyse a number of different conditions in one single experiment. The method is particularly useful for studying the expression profile of a sample obtained from different disease states. Samples which are analysed are derived from one or more of a healthy person, a person with a benign disorder, a person with a malignant disorder (mild or agressive), from persons responding or not responding to a treatment, from persons having a disorder which manifests in different parts of the body, from persons before during and after treatment, from persons receiving a different method of treatment and from otherwise healthy persons having one or more symptoms of a disorder. Disorders which are considered in the context of the present invention include bacterial or viral infections, immunological disorders, cardiovascular disorders and cancer.

Other arrangements of the systems and methods embodying the invention will be obvious for those skilled in the art.

It is to be understood that although preferred embodiments, specific constructions and configurations, as well as materials, have been discussed herein for devices according to the present invention, various changes or modifications in form and detail may be made without departing from the scope and spirit of this invention.

### EXAMPLES

### Example 1: Determination of relative expression levels of individual peptides using isotopic and isobaric labelling.

Using labelling reagents comprising unique combinations of isobaric and isotopic label components it is possible to determine the relative amount of an individual peptide within a mixture of labelled peptides with different isobaric and isotopic labelling combinations. This is exemplified by the following theoretical example. An internal peptide 8 samples (1 to 8) is labelled with a combination of isotopic labels a or b and isobaric labels A, B, C and D according to the scheme in Table 4.

**Table 4: Determination of relative concentrations of a labelled polypeptide in a mixture**

| | | | | | | | | |
|---|---|---|---|---|---|---|---|---|
| peptide | 1 | 2 | 3 | 4 | 5 | 6 | 7 | 8 |
| Isotopic label | a | a | a | a | b | b | b | b |
| Isobaric label | A | B | C | D | A | B | C | D |
| pooled sample | 1aA, 2aB, 3aC, 4aD, 5bA, 6bB, 7bC, 8bD | | | | | | | |
| First separation MS | 1aA, 2aB, 3aC, 4aD | | | | 5bA, 6bB, 7bC, 8bD | | | |
| Ratio isotopic label | 2 | | | | 3 | | | |
| Second separation MS/MS | 1aA | 2aB | 3aC | 4aD | 5bA, | 6bB, | 7bC, | 8bD |
| Ratio isobaric label | 1 | 9 | 3 | 7 | 1 | 8 | 6 | 5 |
| Individual ratio | 1/20 x 2/5 = 0.02 | 9/20 x 2/5 = 0.18 | 3/20 x 2/5 = 0.06 | 7/20 x 2/5 = 0.14 | 1/20 x 3/5 = 0.03 | 8/20 x 3/5 = 0.24 | 6/20 x 3/5 = 0.18 | 5/20 x 3/5 =0.15 |
| Normalised | 1 | 9 | 3 | 7 | 1.5 | 12 | 9 | 7.5 |

Thereafter all samples are pooled and subjected to chromatography wherein labelled peptides behave in the same manner regardless from the particular isotopic/isobaric label component combination on a peptide. The differently labelled versions of the peptide elute as one fraction. The fraction is applied on an MS where it will separate in a fraction with high isotope and a fraction with light isotope. Of these two fractions the relative amount can be calculated. In Table 4, 50 % more peptide with the b isotope is measured than with the a isotope. Subsequently, the two peptide fractions with different isotopic labels are fragmented which allows to determine the relative ratio of isobaric peptides within each peptide fraction with a given isotopic label (theoretical ratio's indicated in Table 4).

## Claims

1. A set of labelling reagents for mass spectrometry analysis of polypeptides, each labelling reagent comprising:
- an allobaric label component,
- a protein/peptide reactive group, and
- an isobaric label component comprising a reporter group (RP) and a balance group (BG).

2. The set of labelling reagents according to claim 1, wherein the allobaric label component is an isotopic label component.

3. The set of labelling reagents according to claim 1, wherein the allobaric label component is comprised within the reporter and/or the balance group of the isobaric label.

4. The set of labelling reagents of claim 1 wherein the protein/peptide reactive group reacts with a cysteine in a protein or polypeptide.

5. The set of labelling reagents of claim 1, wherein each labelling reagent further comprises an affinity tag.

6. The set of labelling reagents according to claim 5, wherein the affinity tag is cleavable.

7. The reagent according to claim 5, wherein the affinity tag is biotin.

8. A method for simultaneously analysing the presence of one or more polypeptides comprising a functional group in different samples, which comprises the steps of:
a) a labelling step wherein each sample is labelled with one of a set of differential double labelling reagents, by allowing the labelling reagents to react with a functional group of the one or more polypeptides in the samples, or peptides generated therefrom;
wherein the set differential double labelling reagents have the same or essentially the same chemical structure and comprise an allobaric and an isobaric label component and wherein each labelling reagent comprises a unique combination of an allobaric and an isobaric label component,
b) pooling the different samples to obtain a polypeptide sample mix,
c) selectively isolating the double labelled polypeptides or peptides from the polypeptide sample mix; and
d) analysing the relative occurrence of the isolated labelled polypeptides or peptides by Mass Spectroscopy,

9. The method according to claim 8, wherein the allobaric labelling components are isotopic labelling components

10. The method according to claim 8, wherein the labelling reagents of the set of labelling reagents comprise an affinity tag.

11. The method according to claim 8, which further comprises after step (a) the step of cleaving the proteins into peptides.

12. The method according to claim 11, wherein the cleavage is performed with trypsin.

13. The method according to claim 8, wherein in step c) the polypeptides or peptides are selectively isolated based on the affinity tag present on said labelled polypeptides or peptides.

14. The method according to claim 8, wherein the functional group is a thiol.

15. The method according to claim 8, wherein the functional group of the polypeptides or peptides generated therefrom are present as a result of a modification of another functional group.

16. The method of claim 8, wherein step (c) comprises the step of isolating a labelled polypeptide or peptide fraction from said pooled polypeptides by electrophoresis or chromatography.

17. The method of claim 8, wherein step (d) comprises the step of determining with MS the relative amount of different masses in said isolated labelled polypeptide or peptide fraction obtained in step (c).

18. The method of claim 17, wherein step (d) further comprises the step of subjecting said isolated labelled polypeptide fraction to collision-induced dissociation (CID) and determining the relative amount of different isobarically labelled polypeptides therein.

19. The method of claim 18, wherein the relative amount of different isobarically labelled polypeptide is determined by determining the relative amount of reporter groups released from the isobaric label after CID.

20. The method of claim 19, further comprising the step of determining the sequence of said polypeptide.

21. A method for labelling a polypeptide sample with a labelling reagent according to claim 1 having an isobaric and an allobaric label component comprising the step of reacting said labelling reagent with a functional group on the polypeptides in said sample.

22. Use of the method of according to any of claims 8 to 21 for identifying proteins which are differentially expressed between a protein sample of a control and one or more experimental or disease protein samples.

23. A mixture of polypeptides or peptides each comprising, a label comprising an isotopic label component and isobaric label component, which label has been linked to the polypeptides or peptides through a functional group in the polypeptides or peptides.

24. The mixture of polypeptides or peptides of claim 23 wherein the functional group is selected from the group consisting of:
- a primary amine on the N-terminus or on Lysine,
- a carboxylgroup on Aspartic acid, Glutamic acid or the C-terminus, and
- a thiol group on Cysteine.

25. The mixture of peptides according to claim 23, comprising peptides which are tryptic peptides of proteins.

26. The mixture according to claim 23, wherein the number of different labels present on the polypeptides or peptides is at least 4.

27. A kit comprising four or more labelling reagents, each labelling reagent comprising an isobaric label component, an allobaric label component and a protein/peptide reactive group, wherein each labelling reagent in said kit has the same or essentially the same chemical structure and wherein each labelling reagent within said kit comprises a unique combination of said allobaric and said isobaric label component.

28. The kit according to claim 27 wherein said labelling reagents comprise an affinity tag.

29. A method for determining relative amounts of individual polypeptides or peptides with the same amino acid sequence in a pooled mixture of these, wherein each individual polypeptide or peptide, prior to pooling, has been labelled with one of a set of differential double labelling reagents, whereby the set of differential double labelling reagents have the same or essentially the same chemical structure and carry an isotopic and an isobaric label component and each labelling reagent comprises a unique combination of different isotopic and an isobaric label components, comprising the steps of
a) optionally isolating the polypeptides or peptides by liquid chromatography, so as to obtain an isolated fraction comprising said individual polypeptides or peptides having the same amino acid sequence,
b) separating the peptides or polypeptides in the pooled mixture or the isolated fraction of polypeptides obtained in step (a) into further fractions of polypeptides or peptides with different mass, by a first MS and determining the relative amount of each peptide fraction with a different mass.
c) Subjecting the peptide fractions obtained in step (b) to a second MS, under conditions wherein the isobaric label components of the individual polypeptides are fragmented and determining the amounts of each of the fragmented isobaric label components of the individual peptides within the peptide fractions.
d) determining from the amount of each of the isobaric label components determined in step (c), the relative amount of the individual peptides within a peptide fraction obtained in step (b),
e) calculating from the relative amounts of each fraction with a different mass determined in step (b) and from the relative amounts of the individual peptides determined in step (d), the relative amount of each individual polypeptide having the same amino acid sequence present in said pooled mixture.

30. A device (100) for multiplex labelling and analysis of protein samples comprising at least two sources of samples (101), a labelling unit (103) and corresponding label sources (104) each comprising a labelling reagent comprising:
- an allobaric label component,
- a protein/peptide reactive group, and
- an isobaric label component comprising a reporter group (RP) and a balance group (BG),
and further comprising a separation unit (107), a mass spectrometer unit (108) and a data analysis unit (109).

31. The device according to claim 30, further comprising one or more elements selected from the group consisting of a sample preparation unit (102), a protein cleavage unit (105) and an affinity purification unit (106).
